(19) Europäisches Patentamt
European Patent Office
Office européen des brevets

(11) EP 1 633 779 B1

(12) # EUROPÄISCHE PATENTSCHRIFT

(45) Veröffentlichungstag und Bekanntmachung des Hinweises auf die Patenterteilung:
**13.10.2010 Patentblatt 2010/41**

(21) Anmeldenummer: **04735228.1**

(22) Anmeldetag: **28.05.2004**

(51) Int Cl.:
***C07K 14/39*** *(2006.01)* ***C12P 41/00*** *(2006.01)*
***C12P 7/00*** *(2006.01)* ***C07K 16/40*** *(2006.01)*

(86) Internationale Anmeldenummer:
**PCT/EP2004/005831**

(87) Internationale Veröffentlichungsnummer:
**WO 2004/111083 (23.12.2004 Gazette 2004/52)**

(54) **OXIDOREDUKTASE AUS PICHIA CAPSULATA**

OXIDOREDUCTASE FROM PICHIA CAPSULATA

OXYDOREDUCTASE EXTRAITE DE PICHIA CAPSULATA

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **18.06.2003 DE 10327454**

(43) Veröffentlichungstag der Anmeldung:
**15.03.2006 Patentblatt 2006/11**

(73) Patentinhaber: **IEP GmbH**
**65203 Wiesbaden (DE)**

(72) Erfinder:
- **GUPTA, Antje**
  **65207 Wiesbaden (DE)**
- **ZIMMER, Anke**
  **65347 Eltville (DE)**
- **BOBKOVA, Maria**
  **65510 Idstein (DE)**

(74) Vertreter: **Schwarz, Albin et al**
**Schwarz & Partner**
**Patentanwälte**
**Wipplingerstraße 30**
**1010 Wien (AT)**

(56) Entgegenhaltungen:
**WO-A-93/18138 WO-A-02/086126**

- **JONES J B: "HORSE LIVER ALCOHOL DEHYDROGENASE AN ILLUSTRATIVE EXAMPLE OF THE POTENTIAL OF ENZYMES IN ORGANIC SYNTHESIS" CHIBATA, I., S. FUKUI AND L. B. WINGARD, JR. (ED.). ENZYME ENGINEERING, VOL. 6. INTERNATIONAL ENZYME ENGINEERING CONFERENCE, KASHIKOJIMA, JAPAN, SEPT. 20-25, 1981. XXII+538P. PLENUM PRESS: NEW YORK, N.Y., USA; LONDON, ENGLAND. ILLUS SERIES : ENZYME E, 1982, Seiten 107-116, XP009039464 ISSN: 0-306-41121-0 in der Anmeldung erwähnt**
- **XIE SHENG-XUE ET AL: "NAD+-dependent (S)-specific secondary alcohol dehydrogenase involved in stereoinversion of 3-pentyn-2-ol catalyzed by Nocardia fusca AKU 2123" BIOSCIENCE BIOTECHNOLOGY AND BIOCHEMISTRY, Bd. 63, Nr. 10, Oktober 1999 (1999-10), Seiten 1721-1729, XP009039491 ISSN: 0916-8451 in der Anmeldung erwähnt**
- **SCHÜTTE H ET AL: "Purification and characterization of a nicotinamide adenine dinucleotide-dependent secondary alcohol dehydrogenase from Candida boidinii." BIOCHIMICA ET BIOPHYSICA ACTA. 16 JUN 1982, Bd. 716, Nr. 3, 16. Juni 1982 (1982-06-16), Seiten 298-307, XP001203741 ISSN: 0006-3002 in der Anmeldung erwähnt**
- **CANNIO RAFFAELE ET AL: "The alcohol dehydrogenase gene: Distribution among Sulfolobales and regulation in Sulfolobus solfataricus" FEMS MICROBIOLOGY LETTERS, Bd. 170, Nr. 1, 1. Januar 1999 (1999-01-01), Seiten 31-39, XP002304730 ISSN: 0378-1097 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 633 779 B1

- BAYER MANFRED ET AL: "Purification and characterization of the NADH-dependent (S)-specific 3-oxobutyryl-CoA reductase from Clostridium tyrobutyricum" ARCHIVES OF MICROBIOLOGY, Bd. 163, Nr. 4, 1995, Seiten 310-312, XP009039585 ISSN: 0302-8933

## Beschreibung

Die vorliegende Erfindung betrifft eine Oxidoreduktase, ein Verfahren zur enantioselektiven Reduktion von Carbonylverbindungen zu den entsprechenden (S)-Hydroxyverbindungen und ein Verfahren zur Gewinnung der chiralen (R)-Hydroxyverbindung.

Optisch aktive Hydroxyverbindungen sind wertvolle chirale Bausteine mit breiter Anwendung für die Synthese von pharmakologisch wirksamen Verbindungen, aromatischer Substanzen, Pheromonen, Agrochemikalien oder Enzyminhibitoren.

Die Zahl der für großtechnische Anwendungen in der Biokatalyse geeigneter und in ausreichendem Maße preiswert zur Verfügung stehenderCarbonylreduktasen istdabei sehr begrenzt. Die folgenden NADH abhängigen S-spezifischen Carbonyl-reduktasen sind bekannt:

Alkoholdehydrogenase aus Pferdeleber (HLADH) (Enzyme Engineering, Vol 6, 1982, Seite 107).
Alkoholdehydrogenase aus Hefe (YADH) (Alcohol dehydrogenases: The Enzymes, (1963) Seiten 25-83. New York: Academic Press),
Carbonylreduktase aus Candida parapsilosis (CPCR) (US 5,523,223 und US 5,763,236),
Carbonylreduktase aus Rhodococcus erythropolis (RECR) (US 5,523,223) und Norcardia fusca (Biosci. Biotechnol. Biochem.,63 (10) (1999), Seiten 1721-1729), Alkohodehydrogenase aus Candida boidinii (Biochim,. Biophys. Acta 716, (1982), Seiten 298-307) oder
Alkoholdehydrogenase aus Sulfolobus solfataricus (FEMS Microbiology Letters, 170 (1999), Seiten 31-39).

Keine der genannten Carbonylreduktasen hat bisher großtechnisch Anwendung gefunden. Der Hauptgrund hierfürist neben den oft zu engen Substratspektren oder der geringen Enantioselektivität der Enzyme vor allem die Verfügbarkeit dieser Enzyme. Die meisten der genannten Enzyme konnten bisher nicht in ausreichendem Maße zu günstigen Preisen zur Verfügung gestellt werden.

Ein weiteres Problem bei der Anwendung der genannten Carbonylreduktasen ist die Regenerierung der Cofaktoren NADH oder NADPH. Die bekannten Verfahren verwenden entweder substratgekoppelte Coenzymregenerierung, beispielsweise mit 2-Propanol, oder enzymgekoppelter Coenzymregenerierung, beispielsweise mit Formiatdehydrogenase.

Nachteil der enzymgekoppelten Coenzymregenerierung mit Formiatdehydrogenase ist deren niedrige spezifische Aktivität (4 bis 10 U/mg), daher ist selbst die rekombinante Formiatdehydrogenase vergleichsweise teuer (J. Biotechnol. Bioeng. [1999] 64, Seiten 187-193).

Nachteil der substratgekoppelten Coenzymregeneration mit Isopropanol ist die ungünstige Gleichgewichtslage und die unzureichende Enzymstabilität gegenüber den verwendeten Cosubstraten wie Isopropanol.

Aufgabe der Erfindung ist es, eine Oxidoreduktase zur Verfügung zu stellen, die sich durch ein breites Substratspektrum, hohe Enantioselektivität und durch hohe Stabilität gegenüber organischen Lösungsmitteln auszeichnet.

Diese Aufgabe wird durch eine Oxidoreduktase gelöst, welche in Anwesenheit von NADH und Wasser eine carbonylverbindung zu der entsprechenden (S)-Hydroxyverbindung reduziert, wobei mehr als 70 % der Aminosäuren identisch sind mit der Aminosäuresequenz SEQ ID NO: 9 und sie eine spezifische Aktivität von mehr als 1 μmol pro mg Protein aufweist, bezogen auf die Umsetzung von Ethyl-4-chlor-3-oxobuttersäure zu (R)-Ethyl-4-chlor-3-hydroxybuttersäure.

Es wurde nun gefunden, dass durch eine neue Oxidoreduktase die genannten Nachteile der Verfahren aus dem Stand der Technik behoben werden können.

Die Erfindung betrifft in zweckmäßiger Weise Oxidoreduktasen, die aus Hefen der Gattungen Pichia oder Candida, insbesondere aus Pichia capsulata gewinnbar sind.

Die Erfindung betrifft in weiterer Ausgestaltung eine Nukleinsäuresequenz, die die DNA-Sequenz gemäß SEQ ID NO: 8 aufweist und eine Oxidoreduktase, die Aminosäuresequenz gemäß SEQ ID NO: 9 aufweist. Diese Sequenzen sind im anliegenden Sequenzprotokoll beschrieben.

Bevorzugt ist eine Oxidoreduktase, bei der 80% bis 99,5%, insbesondere 90% bis 99,5 %, speziell 99 % bis 99,5 % der Aminosäuren identisch zu der Aminosäuresequenz von SEQ ID NO: 9 sind. Die Messung der spezifischen Aktivität der Oxidoreduktase gemäß SEQ ID NO: 9 oder seiner wie nachfolgend definierten Derivate oder Analogons erfolgt mit dem Testsystem, das in Beispiel 1 beschrieben wird.

Die Erfindung betrifft desweiteren die Oxidoreduktase, die die Aminosäuresequenz von SEQ ID NO: 9 aufweist und ein-, zwei-, drei-, vier- oder fünffach durch ein wasserlösliches Polymer modifiziert ist. Ein wasserlösliches Polymer ist beispielsweise Polyethylenglycol. Die Bindung des Polyethylenglycols erfolgt bevorzugt am N-terminalen Ende des Proteins gemäß SEQ ID NO: 9. Die Oxidoreduktase gemäß SEQ ID NO: 9 kann auch an einen Festkörper wie Polyethylen, Polystyrol, Polysaccharid, Cellulose oder Cellulosederivate gebunden sein.

Die Erfindung betrifft ferner ein Fusionsprotein, das dadurch gekennzeichnet ist, dass es die Oxidoreduktase mit der Aminosäuresequenz SEQ ID NO:9 darstellt, die mit einem weiteren Polypeptid am N-terminalen oder Carboxy-terminalen Ende über eine Peptidbindung verbunden sind. Fusionsproteine können beispielsweise leichter von anderen Proteinen abgetrennt werden oder werden in einer größeren Menge in den Zellen exprimiert.

Die Erfindung betrifft ferner einen Antikörper, der spezifisch an die Oxidoreduktase gemäß SEQ ID NO: 9 bindet. Die Herstellung dieser Antikörper erfolgt nach bekannten Methoden durch Immunisierung von geeigneten Säugetieren und anschließenderGewinnung der Antikörper. Die Antikörper können monoklonal oder polyklonal sein.

Die Erfindung betrifft desweiteren eine isolierte DNA-sequenz, die für die oben genannte Oxidoreduktase, kodiert, wobei die DNA-Sequenz ausgewählt wird aus der Gruppe, bestehend aus:

a) einer DNA-Sequenz, welche die Nukleotidsequenz gemäß SEQ ID NO: 8 aufweist oder deren komplementären Strang, und
b) einer DNA-Sequenz, welche mit einer DNA-Sequenzen gemäß a) hybridisiert, wobei die Hybridisierung unter stringenten Bedingungen erfolgt.

Die Bedingungen bei der Hybridisierung sind in Sambrok and Russel, Molecular Cloning a laboratory Manual, Vol 1, Chapter 1 Protocol 30-32 beschrieben.

Die Erfindung betrifft ferner einen Klonierungsvektor, enthaltend eine oder mehrere der obengenannten Nukleinsäure- oder DNA-Sequenzen. Die Erfindung betrifft ferner einen Expressionsvektor, der sich in einer Bakterien-, Insekten-, Pflanzen- oder Säugetierzelle befindet und eine oder mehrere der obengenannten Nukleinsäure- oder D NA-Sequenzen enthält, die In geeigneter Weise mit einer Expressionskontrollsequenz verbunden sind. Die Erfindung betrifft ferner eine Wirtszelle, die eine Bakterien-, Hefe-, Insekten-, Pflanzen- oder Säugetierzelle ist und mit einem Expressionsvektor transformiert oder transfektiert wurde.

Dies Identitäten der vorgenannten DNA-Sequenzen oder Aminosäuresequenz werden dadurch berechnet, dass die Anzahl der Aminosäuren oder Nukleinsäurebasen summiert wird, die mit Tellsequenzen der jeweiligen Proteine oder DNA-Sequenzen identisch sind, und die Summe durch die Gesamtzahl der Aminosäuren oder Nukleinsäurebasen dividiert und mit Hundert multipliziert wird.

Geeignete Klonierungsvektoren sind beispielsweise ppCR-Script, pCMV-Script, pBluescript (Stratagene), pDrive cloning Vector (Quiagen, Hilden, Deutschland), pS Blue, pET Blue, pET LIC-Vektoren (Novagen, Madison, USA) sowie TA-PCR Klonierungsvektoren (Invitrogen, Karlsruhe, Deutschland).

Geeignete Expressionsvektoren sind beispielsweise pKK223-3, pTrc99a, pUC, pTZ, pSK, pBluescript, pGEM, pQE, pET, PHUB, pPLc, pKC30, pRM1/pRM9, pTrxFus,pAS1, pGEx, pMAL oder pTrx.

Geeignete Expressionskontrollsequenzen sind beispielsweise trp-lac (tac)-Promotor, trp-lac (trc) promotor, lac-Promotor, T7-Promotor oder ApL-Promotor.

Die Oxidoreduktase aus Pichia capsulata ist ein Homotetramer mit einem Molekulargewicht von 34 $\pm$ 2 kDa, bestimmt im SDS-Gel, und einem Molekulargewicht von 140 $\pm$ 10 kDa, bestimmt mit Gelpermeationchromatographie. Das Temperaturoptimum der Oxidoreduktase liegt im Bereich von 40°C bis 45 °C, das pH-Optimum für die Reduktionsreaktion liegt im Bereich von 6,5 bis 7,0 und das pH-Optimum für die Oxidationsreaktion liegt im Bereich von 7.8 und 8,2. Die Oxidoreduktase aus Pichia capsulata weist eine gute Temperatur- und pH-Stabilität auf und ist im pH-Bereich von 5,5 bis 8,5 und im Temperaturbereich von 15 °C bis 40 °C für 5 Stunden stabil und zeigt ferner eine hohe Stabilität in organischen Lösungsmitteln.

Das Enzym ist insbesondere aus Hefen der Gattung Pichia isolierbar und kann im spektrophotometrischen Test über die Abnahme von NADH bei 340 nm in Gegenwart eines entsprechenden Substrates, beispielsweise Ethyl-4-chloro-3-oxobutyrat oder 2-Butanon, nachgewiesen werden.

Die erfindungsgemäße Oxidoreduktase aus Pichia capsulata wurde kloniert und konnte in Escherichia coli (E. coli) mit Aktivitäten von 1000 bis 10 000 U/g E. coli Feuchtgewicht überexprimiert werden. Das Enzym ist preiswert und in großen Mengen verfügbar. Sequenzvergleiche in Datenbnaken zeigen auf, dass es sich bei der erfindungsgemäße Oxidoreduktase aus Pichia capsulata um eine Zink abhängige Carbonylreduktase handelt.

Die Erfindung betrifft auch ein Verfahren zur Gewinnung der Oxidoreduktase aus Pichia capsulata. Dazu wird die DNA, die für die Oxidoreduktase aus Pichia capsulata kodiert, beispielsweise in einem geeigneten prokaryotischen oder eukaryotischen Mikroorganismus exprimiert. Bevorzugt wird die Oxidoreduktase aus Pichia capsulata in einen E. coli Stamm transformiert und exprimiert, insbesondere in E. coli BL21 star (DE3) Zellen.

Die Oxidoreduktase aus Pichia capsulata läßt sich beispielsweise so gewinnen, dass die oben genannten rekombinanten E. coli Zellen kultiviert werden, die Expression der Oxidoreduktase induziert wird und anschließend nach etwa 10 bis 18 Stunden (h) die Zellen durch Ultraschallbehandlung oder durch Naßvermahlung mit Glasperlen in einer Kugelmühle (Retsch, GmbH, Haan Deutschland 10 min, 24 Hz) aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer Ionenaustauschchromatographie unterworfen, beispielsweise durch Ionenaustauschchromatographie in einem Q-Sepharose Fast Flow ® Gerät (Fa. Pharmacia).

Die Erfindung betrifft ferner ein Verfahren zur enantioselektiven Reduktion von Carbonylverbindungen zu den entsprechenden (S)-Hydroxyverbindungen, das dadurch gekennzeichnet ist, dass

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert, und
b) die gebildete chirale (S)-Hydroxyverbindung isoliert wird.

Das erfindungsgemäße Verfahren weist eine hohe Standzeit auf, eine enantiomeren Reinheit von mehr als 95 % der hergestellten chiralen (S)-Hydroxyverbindungen und eine hohe Ausbeute bezogen auf die eingesetzte Menge der Carbonylverbindung.
Unterdem Begriff "NADH"wird reduziertes Nicotinamid-adenin-dinucleotid verstanden. Unter dem Begriff "NAD" wird Nicotinamid-adenin-dinucleotid verstanden.
Unter dem Begriff "Carbonylverbindung" werden beispielsweise Verbindungen der Formel I

R1-C(O)-R2 (I)

verstanden.
Der Rest R1 steht beispielsweise für

1) -($C_1$-$C_{20}$)-Alkyl, worin Alkyl geradkettig oder verzweigt ist,
2) ($C_2$-$C_{20}$)-Alkenyl, worin Alkenyl geradkettig oder verzweigt ist und je nach Kettenlänge ein, zwei, drei oder vier Doppelbindungen enthält,
3) -($C_2$-$C_{20}$)-Alkinyl, worin Alkinyl geradkettig oder verzweigt ist und gegebenenfalls ein, zwei, drei oder vier Dreifachbindungen enthält,
4) -($C_6$-$C_{14}$)-Aryl,
5) -($C_1$-$C_8$)-Alkyl-($C_6$-$C_{14}$)-Aryl,
6) -($C_5$-$C_{14}$)-Heterocyclus, der unsubstituiert oder ein- bis dreifach substituiert ist durch Halogen, Hydroxyl, Amino oder Nitro, oder
7) -($C_3$-$C_7$)-Cycloalkyl,

wobei die unter 1) bis 7) genannten Reste unsubstituiert sind oder ein-, zwei- oder dreifach substituiert sind, unabhängig voneinander, durch

a) -OH,
b) Halogen, wie Fluor, Chlor, Brom oder Jod,
c) -$NO_2$ oder
d) -$NH_2$.

Der Rest R2 steht beispielsweise für

1) -($C_1$-$C_6$)-Alkyl, worin Alkyl geradkettig oder verzweigt ist,
2) -($C_2$-$C_6$)-Alkenyl, worin Alkenyl geradkettig oder verzweigt ist und je nach Kettenlänge ein, zwei oder drei Doppelbindungen enthält,
3) -($C_2$-$C_6$)-Alkinyl, worin Alkinyl geradkettig oder verzweigt ist und gegebenenfalls ein oder zwei Dreifachbindungen enthält, oder
4) -($C_0$-$C_{10}$)-Alkyl-C(O)-O-($C_1$-$C_6$)-Alkyl, worin Alkyl gerade oder verzweigt ist und unsubstituiert oder ein- bis dreifach substituiert ist durch Halogen, Hydroxyl, Amino oder Nitro,

wobei die unter 1) bis 4) genannten Reste unsubstituiert sind oder ein-, zwei- oder dreifach substituiert sind unabhängig voneinander durch

a) -OH,
b) Halogen, wie Fluor, Chlor, Brom oder Jod,
c) -$NO_2$ oder
d) -$NH_2$.

Unter dem Begriff chirale "(S)-Hydroxyverbindung" werden beispielsweise Verbindungen der Formel II

R1-C(OH)-R2 (II)

verstanden, wobei die -OH Gruppe in der Regel in (S)-Konfiguration zum Kohlenstoffatom steht, an das sie gebunden ist und R1 und R2 die Bedeutung wie in Formel I haben.

Sollte jedoch in der Nähe des Alkohols eine Carbonylgruppe oder ein Halogenatom stehen, ändert sich die Nomenklatur und die enantioselektiven Alkohole werden dann auch als (R)-Alkohole bezeichnet. Dies ist jedoch nur eine Frage der Nomenklatur, aber ändert nichts an der stereoselektiven Art und Weise wie die erfindungsgemäße Oxidoreduktase die Reduktion durchführt.

Unter dem Begriff Aryl werden aromatische Kohlenstoffreste verstanden mit 6 bis 14 Kohlenstoffatomen im Ring. -($C_6$-$C_{14}$)-Arylreste sind beispielsweise Phenyl, Naphthyl, 1-Naphthyl, 2-Naphthyl, Biphenylyl, 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-($C_1$-$C_{20}$)-Alkyl wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl. Unter dem Begriff "-$C_0$-Alkyl" wird eine kovalente Bindung verstanden.

Unter dem Begriff "-($C_3$-$C_7$)-Cycloalkyl" werden cyclische Kohlenwasserstoffreste verstanden wie Cyclopropyl, Cylobutyl, Cyclopentyl, Cyclohexyl oder Cycloheptyl.

Der Begriff "-($C_5$-$C_{14}$)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe -($C_5$-$C_{14}$)-Heterocyclus sind Reste, die sich von Pyrrol, Furan, Thiophen, Imidazol, Pyrazol, Oxazol, Isoxazol, Thiazol, Isothiazol, Tetrazol,1,2,3,5-Oxathiadiazol-2-Oxide, Triazolone, Oxadiazolone, Isoxazolone, Oxadiazolidindione, Triazole, welche durch F, -CN, -$CF_3$ oder -C(O)-O-($C_1$-$C_4$)-Alkyl substituert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benzanellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methylimidazolyl, zum Beispiel 1-Methyl-2-, -4-oder-5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl,4,5,6,7-Tetrahydro-2-indolyl,Cyclohepta[b]-5-pyrrolyl,2-, 3-oder4-Chinolyl,1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2-oder3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind beispielsweise Ethyl-4-chloracetoacetat, Methylacetoacetat, Ethyl-8-chloro-6-oxooctansäure, Ethyl-3-oxovaleriat, 4-Hydroxy-2-butanon, Ethyl-2-oxovaleriat, Ethyl-2-oxo-4-phenylbuttersäure, Ethylpyruvat, Ethylphenylglyoxylat, 1-Phenyl-2-propanon, 2,3-Dichloracetophenon, Acetophenon, 2-Octanon, 3-Octanon oder 2-Butanon.

Die entsprechend gebildeten S-Alkohole sind beispielsweise (R)-Ethyl-4-chloro-3-hydroxybuttersäure, Ethyl-(S)-2-Hydroxy-4-phenylbuttersäure, (S)-2-Octanol oder (R)-Ethyl-8-chlor-6-hydroxyoctansäure.

Geeignete Oxidoreduktasen stammen beispielsweise aus Pichia capsulata. Die Oxidoreduktase kann in dem erfindungsgemäßen Verfahren entweder vollständig gereinigt oder teilweise gereinigt eingesetzt werden. Das Verfahren wird mit der erfindungsgemäßen Oxidoreduktase oder mit Zellen, enthaltend die erfindungsgemäße Oxidoreduktase, durchgeführt. Die eingesetzten Zellen können dabei nativ, permeabilisiert oder lysiert vorliegen. Bevorzugt wird die klonierte Oxidoreduktase gemäß SEQ ID NO: 9 eingesetzt.

Die Volumenaktivität der eingesetzten Oxidoreduktase beträgt von 100 Units/ml (U/ml) bis 5000 U/ml, bevorzugt etwa 500 U/ml.

Je kg umzusetzender Verbindung der Formel I werden 5000 bis 2 000 000 U Oxidoreduktase eingesetzt, bevorzugt etwa 10 000- 200 000 U. Der Enzymeinheit 1 U entspricht dabei der Enzymmenge die benötigt wird um 1 µmol der Verbindung der Formel I je Minute (min) umzusetzen.

Die Erfindung betrifft ferner ein Verfahren zur enantioselektiven Reduktion von Carbonylverbindungen zu den entsprechenden (S)-Hydroxyverbindungen, bei dem

a) eine Carbonylverbindung in Anwesenheit der erfindungsgemäßen Oxidoreduktase, NADH und Wasser zur entsprechenden (S)-Hydroxyverbindung reduziert wird,
b) das durch die Oxidoreduktase gebildete NAD mit einem Cosubstrat zu NADH reduziert und
c) die gebildete chirale (S)-Hydroxyverbindung isoliert wird.

Die eingesetzten Mengen der Carbonylverbindungen und Oxidoreduktasen entsprechen den zuvor genannten Mengen in den beschriebenen Verfahren. Geeignete Cosubstrate für das erfindungsgemäße Verfahren sind Alkohole wie Ethanol, 2-Propanol (Isopropanol), 2-Butanol, 2-Pentanol oder 2-Octanol. Diese Cosubstrate werden mit Hilfe der erfindungsgemäßen Oxidoreduktase und NAD zu den entsprechenden Ketonen und NADH umgesetzt. Dadurch kommt es zur Regenerierung des NADH's.

Die Menge des Cosubstrats für die Regenerierung von NAD zu NADH wie Isopropanol beträgt von 5 % bis 50 % bezogen auf das Gesamtvolumen, bevorzugt von 8 % bis 20 %, insbesondere von 10 % bis 15 %.

Die Erfindung betrifft desweiteren ein Verfahren zur enantioselektiven Gewinnung von (S)-Hydroxyverbindungen, bei dem

a) eine Carbonylverbindung in Anwesenheit der erfindungsgemäßen Oxidoreduktase, NADH und Wasser zur entsprechenden (S)-Hydroxyverbindung reduziert wird,
b) das durch die Oxidoreduktase gebildete NAD mit einer Dehydrogenase und einem Cosubstrat zu NADH reduziert und
c) die gebildete chirale (S)-Hydroxyverbindung isoliert wird.

Geeignete Dehydrogenasen sind beispielsweise NADH abhängige Alkohol-Dehydrogenasen aus Bäckerhefe, aus Candida boidinii oder Candida parapsilosis. Geeignete Cosubstrate für die eingesetzte Alkohol-Dehydrogenase sind Alkohole wie Ethanol, 2-Propanol (Isopropanol), 2-Butanol, 2-Pentanol oder 2-Octanol.

Ferner kann die NAD-Reduktion auch mittels Formiat-Dehydrogenase (Tishkov et al., J. Biotechnol. Bioeng. [1999] 64, 187-193, Pilot-scale production and isolation of recombinant NAD and NADP specific formate dehydrogenase) durchgeführt werden. Geeignete Cosubstrate der Formiat-Dehydrogenase sind beispielsweise Salze der Ameisensäure wie Ammoniumformiat, Natriumformiat oder Calciumformiat.

Bevorzugt kommt die substratgekoppelte Coenzymregenerierung mit einem sekundären Alkohol wie Ethanol, 2-Propanol (Isopropanol), 2-Butanol, 2-Pentanol oder 2-Octanol zur Anwendung. Daher wird das Verfahren bevorzugt ohne eine zusätzliche Dehydrogenase durchgeführt.

Dem im Verfahren eingesetzten Wasser wird bevorzugt ein Puffer zugesetzt, beispielsweise Kaliumphosphat-, Tris/HCl- oder Triethanolamin-Puffer mit einem pH-Wert von 5 bis 10, vorzugsweise einem pH-Wert von 6 bis 9. Die Pufferkonzentration beträgt von 10 mM bis 150 mM.

Der Puffer kann zusätzlich noch Ionen zur Stabilisierung oder Aktivierung der Enzyme enthalten, beispielsweise Zinkionen oder Magnesiumionen.

Die Temperatur beträgt beispielsweise 10 °C bis 60 °C, bevorzugt 30 °C bis 55 °C.

Die Erfindung betrifft desweiteren ein Verfahren zur enantioselektiven Gewinnung von (S)-Hydroxyverbindungen, bei dem

a) eine Carbonylverbindung in Anwesenheit der Oxidoreduktase, NADH und Wasser zur entsprechenden (S)-Hydroxy-verbindung reduziert wird,
b) die Reaktionen in Anwesenheit eines organischen Lösungsmittel durchgeführt und
c) die gebildete chirale (S)-Hydroxyverbindung isoliert wird.

Die bevorzugten organischen Lösungsmittel sind beispielsweise Diethylether, tertiär Butylmethylether, Diisopropylether, Dibutylether, Butylacetat, Heptan, Hexan oder Cyclohexan.

Der Reaktionsansatz besteht beim Einsatz zusätzlicher Lösungsmittel aus einer wässrigen Phase und einer organischen Phase. Die organische Phase wird durch ein geeignetes Lösungsmittel, in dem das Substrat gelöst vorliegt, oder durch das wasserunlösliche Substrat selbst gebildet.

Die organische Phase beträgt dabei 5 % bis 80 % des gesamten Reaktionsvolumens, bevorzugt 10 % bis 40 %.

Das Wasser bildet im erfindungsgemäßen Zwei-Phasen-System die eine flüssige Phase und das organische Lösungsmittel bildet die zweite flüssige Phase. Gegebenenfalls kann auch noch eine feste oder weitere flüssige Phase vorliegen, die beispielsweise durch nicht vollständig gelöste Oxidoreduktase und/oder hinzugefügte Enzyme oder durch die Carbonylverbindung entsteht. Bevorzugt sind jedoch zwei flüssige Phasen ohne feste Phase. Die zwei flüssigen Phasen werden bevorzugt mechanisch gemischt, so dass große Oberflächen zwischen den beiden flüssigen Phasen ausgebildet werden.

Die Konzentration des Cofaktors NADH bezogen auf die wässrige Phase beträgt 0,01 mM bis 1 mM, insbesondere 0,05 mM bis 0,2 mM.

Die Carbonylverbindung wird im erfindungsgemäßen Verfahren in einer Menge von 3 % bis 30 % bezogen auf das Gesamtvolumen eingesetzt, bevorzugt von 5 % bis 15 %, insbesondere von 10 %.

Die Erfindung betrifft ferner ein Verfahren zur enantioselektiven Reduktion von Carbonylverbindungen zu den entsprechenden (S)-Hydroxyverbindungen, bei dem

a) eine Carbonylverbindung in Anwesenheit der Oxidoreduktase, NADH und Wasser zur entsprechenden (S)-Hydroxy-verbindung reduziert wird,
b) die Reaktionen in Anwesenheit eines organischen Lösungsmittel durchgeführt wird,
c) das durch die Oxidoreduktase gebildete NAD mit einem Cosubstrat zu NADH reduziert und
d) die gebildete chirale (S)-Hydroxyverbindung isoliert wird.

Die eingesetzten Mengen der Carbonylverbindungen und Oxidoreduktasen entsprechen den zuvorgenannten Verfahren. Geeignete Cosubstrate für das Verfahren sind Alkohole wie Ethanol, 2-Propanol (Isopropanol), 2-Butanol, 2-Pentanol oder 2-Octanol. Diese Cosubstrate werden mit Hilfe der Oxidoreduktase und NAD zu den entsprechenden Ketonen und NADH umgesetzt. Dadurch kommt es zur Regenerierung des NADH's.
Die Menge des Cosubstrats wie Isopropnaol für die Regenerierung von NAD zu NADH beträgt 5 % bis 50 %, bezogen auf das Gesamtvolumen, bevorzugt 8 % bis 20 % und insbesondere 10 % bis 15 %.
Die Erfindung betrifft desweiteren ein Verfahren zur enantioselektiven Reduktion von Carbonylverbindungen zu den entsprechenden (S)-Hydroxyverbindungen, bei dem

a) eine Carbonylverbindung in Anwesenheit der Oxidoreduktase, NADH und Wasser zur entsprechenden (S)-Hydroxyverbindung reduziert wird,
b) das durch die Oxidoreduktase gebildete NAD mit einer Dehydrogenase und einem Cosubstrat gleichzeitig zu NADH reduziert,
c) die Reaktionen in Anwesenheit eines organischen Lösungsmittel durchgeführt und
d) die gebildete chirale (S)-Hydroxyverbindung isoliert wird.

Bevorzugt wird im erfindungsgemäßen Verfahren noch ein weiterer Stabilisator der Alkohol-Dehydrogenase eingesetzt. Geeignete Stabilisatoren sind beispielsweise Glycerin, Sorbitol, 1,4 -DL-Dithiothreit (DTT) oder Dimethylsulfoxid (DMSO).
Das erfindungsgemäße Verfahren wird beispielsweise in einem geschlossen Reaktionsgefäß aus Glas oder Metall durchgeführt. Dazu werden die Komponenten einzeln in das Reaktionsgefäß übergeführt und unter einer Atmosphäre von beispielsweise Stickstoff oder Luft gerührt. Je nach Substrat und eingesetzter Carbonylverbindung beträgt die Reaktionszeit 1 Stunde bis 48 Stunden, insbesondere 2 Stunden bis 24 Stunden.
Anschließend wird das Reaktionsgemisch aufgearbeitet. Dazu wird die wässrige Phase abgetrennt und die organische Phase gefiltert. Die wässrige Phase kann gegebenenfalls noch einmal extrahiert werden und wie die organische Phase weiter aufgearbeitet werden. Danach wird gegebenenfalls das Lösungsmittel aus der gefilterten organischen Phase verdampft. Auf diese Weise wird das Produkt (R)-Ethyl-4-chlor-3-hydroxybuttersäure mit einer Enantiomerenreinheit von mehr als 99 % und im wesentlichen frei vom Edukt Ethyl-4-chloracetoacetat erhalten. Die Gesamtausbeute der Prozesses beträgt nach Destillation des Produktes 50 % bis 95 %, bezogen auf die eingesetzte Eduktmenge.
Die Erfindung betrifft ferner ein Verfahren zur Gewinnung von chiralen (R)-Hydroxyverbindungen der Formel II,

R1-C(OH)-R2 (II)

mit den Resten R1 und R2 wie sie zuvor definiert sind. Bei diesem Verfahren wird

a) ein Gemisch, enthaltend die racemische Verbindung der Formel II, mit der erfindungsgemäßen Oxidoreduktase, NAD und Wasser inkubiert und
b) die verbliebene chirale (R)-Hydroxyverbindung der Formel II isoliert.

Hierbei wird die (S)-Hydroxyverbindung der Formel II zur entsprechenden Ketoverbindung und NADH umgesetzt.
Die Erfindung betrifft desweiteren ein Verfahren zur Gewinnung von chiralen (R)-Hydroxyverbindungen der Formel II, bei dem

a) ein Gemisch, enthaltend die racemische Verbindung der Formel II, mit der erfindungsgemäße Oxidoreduktase, NAD und Wasser inkubiert wird,
b) das durch die Oxidoreduktase gebildete NADH mit einem Cosubstrat zu NAD oxidiert und
c) die verbliebene chirale (R)-Hydroxyverbindung der Formel II isoliert wird.

Die Erfindung betrifft ferner ein Verfahren zur Gewinnung von chiralen (R)-Hydroxyverbindungen der Formel II, bei dem

a) ein Gemisch, enthaltend die racemische Verbindung der Formel II, mit der erfindungsgemäße Oxidoreduktase, NAD und Wasser inkubiert wird,
b) das durch die Oxidoreduktase gebildete NADH mit einer Dehydrogenase und einem Cosubstrat zu NAD oxidiert und
c) die verbliebene chirale (R)-Hydroxyverbindung der Formel II isoliert wird.

Die Erfindung betrifft in weiterer Ausgestaltung ein Verfahren zur Gewinnung von chiralen (R)-Hydroxyverbindungen der Formel II, bei dem

a) ein Gemisch, enthaltend die racemische Verbindung der Formel II, mit der erfindungsgemäße Oxidoreduktase, NAD und Wasser inkubiert wird,
b) die Reaktionen in Anwesenheit eines organischen Lösungsmittels durchgeführt und
c) die verbliebene chirale (R)-Hydroxyverbindung der Formel II isoliert wird.

Ein weiteres erfindungsgemäßes Verfahren zur Gewinnung von chiralen (R)-Hydroxyverbindungen der Formel II zeichnet sich dadurch aus, dass

a) ein Gemisch, enthaltend die racemische Verbindung der Formel II, mit der erfindungsgemäße Oxidoreduktase, NAD und Wasser inkubiert wird,
b) die Reaktionen in Anwesenheit eines organischen Lösungsmittels durchgeführt,
c) das durch die Oxidoreduktase gebildete NADH mit einer Dehydrogenase und einem Cosubstrat zu NAD oxidiert und
d) die verbliebene chirale (R)-Hydroxyverbindung der Formel II isoliert wird.

Die Reaktionsbedingungen sind im wesentlichen dieselben wie im zuvor genannten Verfahren zur enantiospezifischen Reduktion der Ketoverbindungen der Formel I. Es wird jedoch statt einer enantioselektiven Reduktion der Ketoverbindung der Formel I aus dem racemischen Gemisch der Verbindung der Formel II nur die (S)-Hydroxyverbindung der Formel II enantioselektiv zur entsprechenden Ketoverbindung oxydiert. Dadurch verbleibt die (R)-Hydroxyverbindung der Formel II und kann isoliert werden.
Ferner wird im Verfahren anstelle der als Cosubstrate eingesetzten Alkohole wie Ethanol, 2-Propanol (isopropanol), 2-Butanol, 2-Pentanol oder 2-Octanol, deren entsprechenden Ketone wie Aceton zur Regenerierung des NAD eingesetzt. Beispielsweise wird das Aceton und NADH mit der erfindungsgemäßen Oxidoreduktase oder einer zusätzlichen Dehydrogenase zu NAD und Isopropanol umgesetzt. Die eingesetzten Ketonmengen betragen von 5 % bis 50 % bezogen auf das Gesamtvolumen, bevorzugt 8 % bis 20 % und insbesondere von 10 % bis 15 %.
Die Erfindung wird im folgenden anhand von Beispielen erläutert.

## Beispiele

Beispiel 1: Screening von Hefen nach S-spezifischer Alkoholdehydrogenase

Zum Screening wurde eine Reihe verschiedener Hefestämme in folgendem Medium kultiviert (Angaben jeweils g/l): Hefeextrakt (3), Malzextrakt (3), Peptone (5) und Glucose (10). Das Medium wurde bei 121 °C sterilisiert und die Hefen wurden ohne weitere pH-Regulierung bei 25 °C und auf einem Schüttler bei 160 Umdrehungen pro Minute (rpm) kultiviert. Anschließend wurden 125 mg Zellen mit 800 µl Aufschlusspuffer (100 mM Trieethanolamin (TEA), pH = 7,0) resuspendiert, mit 1 g Glasperlen versetzt und 10 Minuten (min) bei 4 °C in der Kugelmühle aufgeschlossen (Firma Retsch). Der nach 2 min Zentrifugieren mit 12000 rpm erhaltene Überstand (Lysat) wurde im folgenden Aktivitätsscreening und zur Bestimmung des Enatiomerenüberschusses (ee-Wert) eingesetzt. Als Substrate wurden Ethyl-4-chloracetoacetat und 2-Chloro-1-(3-chlorophenyl)ethan-1-on eingesetzt.
Ansatz fürs Aktivitätsscreening:

| | |
|---|---|
| 860 µl | 0,1 M $KH_2PO_4/K_2PO_4$ pH = 7,0 1mM $MgCl_2$ |
| 20 µl | NADPH oder NADH (10 mM) |
| 20 µl | Lysat |
| 100 µl | jeweilige Substrat (100 mM) |

Die Reaktion wurde 1 min bei einer Wellenlänge von 340 nm verfolgt.
Ansatz für ee-Wert Bestimmung:

20 µl Lysat
100 µl NADH oder NADPH (50 mM)
60 µl Substrat (Ethyl-4-chloracetoacetat 100 mM)

Die Ansätze zur ee-Bestimmung wurden nach 24 Stunden (h) mit Chloroform extrahiert und mittels Gaschromatographie (GC) wurde der Enantiomerenüberschuss bestimmt. Der Enantiomerenüberschuss wird wie folgt berechnet:

$$ee(\%) = ((R\text{-Alkohol} - S\text{-Alkohol})/(R\text{-Alkohol} + S\text{-Alkohol})) \times 100.$$

| DSMZ Nr. | Mikrorganismenname | Ethyl-4-chloracetoacetat Aktivität in U/g Zellen Wirtsorganismus | | | |
|---|---|---|---|---|---|
| | | NADH | NADPH | ee- Wert NADH | ee-Wert NADPH |
| 1345 | Yarrowia lipolytika | 0 | 15 | 96 % S | |
| 3434 | Kluyveromyces thermotolerans | 0 | 6 | -- | --- |
| 3435 | Metschnikowia zobelli | 0 | 12 | -- | -- |
| 3795 | Kluyveromyces lactis | 0 | 15 | 80 % S | 90 % S |
| 70130 | Pichia anomala | 0 | 9 | 68 % S | 90 % S |
| 70169 | Pichia membranefaciens | 0 | 8 | -- | ---- |
| 70277 | Pichia angusta | 13 | 4,5 | racemat | 30 % S |
| 70382 | Pichia pastoris | 16 | 16 | 64 % S | 81 % S |
| 70638 | Candida magnoliae | 2,5 | 10 | --- | 80 % R |
| 70125 | Candida parapsilosis | 25 | 11 | 52 % R | 70 % S |
| 2147 | Pichia methanolica | 18 | 27 | 84 % S | 90 % S |
| Candida methylica | | 8 | 13 | 94 % S | 94 % S |
| 70260 | Pichia capsulata | 50 | 5 | 100 % R | |

DSMZ steht für Deutschen Sammlung für Mikroorganismen und Zellkulturen, Mascheroder Weg 1b, 38124 Braunschweig

Definition der Enzymeinheiten: 1 U entspricht der Enzymmenge die benötigt wird um 1 µmol Substrat pro min umzusetzen.

Beispiel 2: Isolierung einer NADH abhängigen (S)-spezifischen Oxidoreduktase aus Pichia capsulata

Zur Isolierung der NADH abhängigen Oxidoreduktase aus Pichia capsulata (P. capsulata) wurde der Mikroorganismus wie unter Beispiel 1 beschrieben kultiviert. Nach Erreichen der stationären Phase wurden die Zellen geerntet und durch Zentrifugieren vom Medium getrennt. Die Enzymfreisetzung erfolgte durch Naßvermahlung mittels Glasperlen, kann aber auch durch andere Aufschlussmethoden erreicht werden. Dazu wurden 100 g P. capsulata mit 400 ml Aufschlusspuffer (100 mM Triethanolamin, 1 mM $MgCl_2$, pH =7,0) suspendiert und mittels einer French press homogenisiert.

Der nach dem Zentrifugieren (7000 rpm) erhaltene Rohextrakt wurde dann mittels FPLC (fast protein liquid chromatography) weiter gereinigt und aufbereitet.

Die erfindungsgemäße Oxidoreduktase konnte in zwei hintereinander folgenden Schritten mittels Ionenaustauschchromatographie an Q-Sepharose Fast Flow (Firma Pharmacia) und Uno Q (Biorad, München, Deutschland) aufgereinigt werden. Dazu wurde das nach dem Zentrifugieren erhaltene Lysat direkt auf eine mit 50 mM Kaliumphosphatpuffer pH = 7,0 äquilibrierte Q-Sepharose FF-Säule aufgetragen und mit steigendem linearen Salzgradienten eluiert. Die Oxidoreduktase wurde dabei bei 0,2 bis 0,3 M NaCl eluiert. Die oxidoreduktase-enthaltenen Fraktionen wurden vereinigt und mittels Ultrafiltration (Ausschlussgrenze 10 kDa) auf ein geeignetes Volumen eingeengt. Anschließend wurde die eingeengten Fraktionen der Oxidoreduktase mittels Uno Q unter Verwendung derselben obengenannten Puffer weiter aufbereitet und gereinigt. Das Enzym wurde dabei bei 0,1 M NaCl eluiert.

Danach wurde das Molekulargewicht der erhaltenen gereinigten Oxidoreduktase mit Hilfe von Gelpermeation (Superdex 200 HR; Pharmacia, 100 mM Triethanolamin, pH = 7, 0,15 M NaCl) bestimmt.

Als Molekulargewichtsstandards wurden Catalase (232 kDa), Aldolase (158 kDa), Albumin (69,8kDa) und Ovalbumin (49,4 kDa) verwendet.

Die folgende Tabelle 2 fasst die erhaltenen Ergebnisse zusammen

Tabelle 2:

| Reinigungsschritt | Volumen [ml] | Aktivität [U/ml] | Gesamtaktivität [U] | Spezifische Aktivität [U/mg] | Ausbeute |
|---|---|---|---|---|---|
| Rohextrakt | 360 | 2,0 | 752 | 0,07 | 100 % |
| Q-Sepharose | 67 | 5,3 | 358 | 5 | 47 % |
| Uno Q | 3,5 | 14 | 50 | 41 | 6,6 % |

Die Enzymaktivität der Oxidoreduktase wurde im Testsystem gemäß Beispiel 1, (Ansatz Aktivitätsscreening) bestimmt und die Bestimmung der Proteinmenge erfolgte gemäß Lowry et al. Journal of Biological Chemistry, 193 (1951): 265-275 oder Peterson et al., Analytical Biochemistry, 100 (1979): 201-220). Der Quotient von Enzymaktivität zu Proteinmenge ergibt die spezifische Aktivität, wobei der Umsatz von 1 μmol pro min 1 Unit (U) entspricht.

Das mittels Gelpermation bestimmte Molekulargewicht der erfindungsgemäßen Oxidoreduktase betrug im nativen Zustand 140 ±10 kDa.

Beispiel 3: Bestimmung der N-terminalen Sequenz der erfindungsgemäßen Oxidoreduktase

Die Enzympräparation nach Beispiel 2 wurde nach der Gelpermeation im 10 % igen Natriumdodecylsulfat (SDS) Gel aufgetrennt und auf eine Polyvinyliden Diflourid-Membran (PVDF-Membran) übertragen.

Die auffällige Bande bei etwa 35 bis 45 kDa wurde einer N-terminalen Sequenzierung mittels Edman-Abbau (Procise 492 (PE-Biosystems) unterworfen. Es wurde folgende N-terminale Sequenz erhalten:

SEQ ID NO: 10

KTQAGYIFKKGA

Beispiel 4: Klonierung der Oxidoreduktase aus Pichia capsulata

Der eukaryotische Mikrorganismus Pichia capsulata gehört zu der Familie Saccaromycetacea. Die Genomstruktur dieses Organismus weist eine Exon-Intron Anordnung auf. Daher wurde für die Identifizierung der Gensequenz, die für die enantioselektive Oxidoreduktase kodiert, eine cDNA Bibliothek aus den aktiven Zellen der Pichia capsulata angelegt.

4.1 Präparation der gesamten RNA aus den Zellen von Pichia capsulata.

600 mg frische Zellen von Pichia capsulata wurden in 2,5 ml eiskalten LETS (10 mM Tris-HCl, pH = 7,4, 10 mM EDTA, 100 mM LiCl, 0,2 % SDS) Puffer resuspendiert. Zu dieser Zellsuspension wurden 5 ml (etwa 20 g) in Salpetersäure gewaschener Glasperlen, die mit 3 ml Phenol (pH 7.0) equilibriert waren, zugegeben. Der gesamte Ansatz wurde dann abwechselnd jeweils 30 Sekunden (sec) geschüttelt (Vortex Genie 2, Scientific Industries Inc., New York, USA) und 30 sec auf Eis gekühlt und insgesamt 10 min behandelt. Anschließend wurden weitere 5 ml eiskalter LETS Puffer dazugegeben. Diese Zellsuspension wurde für 5 min bei 11000 g bei 4 °C zentrifugiert. Die wässrige Phase wurde abgenommen und mit dem gleichen Volumen an Phenol: Chloroform: 3-Methyl-1-butanol (24:24:1) zweimal extrahiert. Anschließend erfolgte eine Extraktion mit Chloroform. Nach der letzten Extraktion wurde die gesamte erhaltene RNA durch die Zugabe von 1/10 Vol von 5 M LiCl bei -20°C für 4 Stunden präzipitiert.

1 mg der isolierten RNA wurde für die m-RNA Gewinnung mittels Oligo-dT Cellulose (mRNA PräpKit, Qiagen) eingesetzt.

Nach der anschließenden Präzipitation wurden 5 μg mRNA für die cDNA Synthese (pBluescript IIXR cDNA Library Construction kit, Stratagene) verwendet. Die nach den Angaben des Herstellers konstruierte Bibliothek wurde in XL-10 Gold E. coli transformiert und auf die Aktivität einer (S)-ADH hin untersucht.

Anhand der Extinktionsabnahme mit NADH als Cofaktor und Ethyl-4-chloraceto-acetat als Substrat wurden zwei Klone (2 /1 und 2 / 2) identifiziert und isoliert. Die Sequenzierung über die multiple Klonierungsstelle, der in den Klonen enthaltenden Plasmide mit Primer T7 (SEQ ID NO: 3) und Primer T3 (SEQ ID NO:4), resultierte in einem Fragment mit der Größe von 1175 bp (SEQ ID NO: 1). Dieses Fragment kodiert für ein Fusionsprotein mit 366 Aminosäuren (SEQ ID NO: 2) und besteht aus dem a-Fragment der β-Galactosidase und der Sequenz der erfindungsgemäßen Oxidoreduktase.

4.2 Synthese eines für eine (S)-ADH aus Pichia capsulata kodierendem Volllänge-Transkript mittels PCR (Polymerase chain reaction)

Basierend auf SEQ ID NO: 1 wurden spezifische Primer für eine nachfolgende Klonierung des Volllänge Transkripts in passende Expressionssysteme konstruiert. Dabei wurden 5'-Primer mit einer Erkennungssequenz für Nde I (bzw. Sph I) und 3'-Primer mit einer Erkennungssequenz für Hind III modifiziert (SEQ ID NO: 5; SEQ ID NO: 6; SEQ ID NO: 7)

Oligo 1-Nde I: 5 '-GGAATTCCATATGTCTGCTCTCTCCAAAAC-3'

Oligo 2-Sph I: 5 '-CACTGCATGCTGATGTCTGCTCTCTCCAAAAC-3'

Oligo 3-Hind III: 5'-CCCAAGCTTTCATGGAAGCATAACCAATCTT-3'

Plasmid DNA isoliert aus dem Klon 2/1 der Expressionsbibliothek von Pichia capsulata diente als Matrize für die Polymerasen Ketten-Reaktion. Die Amplifikation wurde in einem PCR-Puffer [10 mM Tris-HCl, (pH 8,0); 50 mM KCl; 10 mM $MgSO_4$; 1 mM dNTP Mischung (N steht dabei für die Basen A,T, C oder G); je 30 pMol Primer und 2,5 U Platinum Pfx DNA-Polymerase (Invitrogen)] mit 50 ng Template und folgenden Temperatur-Zyklen durchgeführt:

| | |
|---|---|
| Zyklus 1: | 94 ° C, 2 min |
| Zyklus 2 x 30: | 94 ° C, 15 sec |
| | 58 ° C, 30 sec |
| | 68 ° C, 75 sec |
| Zyklus 3: | 68 ° C, 7 min |
| | 4 ° C, ∞ |

Das resultierende PCR-Produkt wurde nach der Reinigung über ein 1 %iges Agarose Gel mit Hilfe von Endonukleasen Nde I und Hind III, oder mit Endonucleasen Sph I und Hind III verdaut, und in das mit gleichen Endonukleasen behandelte Rückgrat des pET21a Vectors (Novagen), oder pQE30 Vectors (Qiagen) ligiert. Nach der Transformation von 2 II des Ligationsansatzes in E. coli Top 10 F' Zellen (Invitrogen) wurden Plasmid-DNA's von Ampicillin-resistenten Kolonien mittels einer Restriktionsanalyse mit Endonukleasen Nde I und Hind III oder Endonucleasen Sph I und Hind III auf das Vorhandensein eines 1100 bp großen Inserts getestet. Der Expressionskonstrukt pET21-PC#10 wurde sequenziert. Das für die erfindungsgemäße Oxidoreduktase kodierende Transkript aus Pichia capsulata besitzt einen offenen Leserahmen von insgesamt 1026 bp (SEQ ID NO: 8), das einem Protein von 341 Aminosäuren entspricht (SEQ ID NO: 9)

4.3 Expression der erfindungsgemäße Oxidoreduktase aus P. capsulata in Star BL 21 (De3) E. coli Zellen

Kompetente Escherichia coli StarBL21 (De3) Zellen (Invitrogen) wurden mit dem für die erfindungsgemäße Oxidoreduktase kodierenden Expressionskonstrukt pET21-PC#10 transformiert.

Der transformierte Stamm wurde in LB Medium (1 % Tryptone, 0,5 % Hefeextrakt, 1 % NaCl) mit Ampicillin (50 ig/ml) kultiviert, bis eine optische Dichte, gemessen bei 500 nm, von 0,5 erreicht wurde. Die Produktion des rekombinanten Oxidoreduktase Proteins wurde durch Zugabe von Isopropylthiogalaktosid (IPTG) in einer Endkonzentration von 1 mM gestartet. Der Induktionsansatz wurde für weitere 15 h bei 25 °C und 220 rpm inkubiert.

Die erreichte Enzymaktivität betrug etwa 6000 U/g Zellfeuchtmasse.

4.4 Expression der erfindungsgemäße Oxidoreduktase aus P. capsulata in RB791 E. coli Zellen

Kompetente Escherichia coli RB791 Zellen wurden mit dem für die erfindungsgemäße Oxidoreduktase Expressionskonstrukt pQE30-PC#12 transformiert.

Der transformierte Stamm wurde in LB Medium (1 % Tryptone, 0,5 % Hefeextrakt, 1 % NaCl) mit Ampicillin (50 ìg/ml) kultiviert, bis eine optische Dichte, gemessen bei 500 nm von 0,5, erreicht wurde. Die Produktion des rekombinanten Oxidoreduktase-Proteins wurde durch Zugabe von IPTG in einer Endkonzentration von 0,1 mM gestartet. Der Induktionsansatz wurde für weitere 15 Stunden bei 25 °C und 220 rpm inkubiert. Die erreichte Enzymaktivität betrug etwa 1000 U/g Zellfeuchtmasse.

Beispiel 5: Charakterisierung der rekombinanten Oxidoreduktase aus P. capsulata

5.1 pH- Optimum

Es wurden die in Tabelle 3 aufgeführten Puffer hergestellt. Die Konzentration der jeweiligen Pufferkomponenten betrug jeweils 50 mM.

Tabelle 3

| pH-Wert | Puffersystem | pH-Wert | Puffersystem |
|---|---|---|---|
| 4 | Na-acetat/Essigsäure | 7,5 | $KH_2PO_4K_2PO_4$ |
| 4,5 | Na-acetat/Essigsäure | 8 | $KH_2PO_4/K_2PO_4$ |
| 5 | Na-acetat/Essigsäure | 8,5 | $KH_2PO_4K_2PO_4$ |
| 5,5 | $KH_2PO_4/K_2PO_4$ | 9 | Glycin/NaOH |
| 6 | $KH_2PO_4/K_2PO_4$ | 9,5 | Glycin/NaOH |
| 6,5 | $KH_2PO_4/K_2PO_4$ | 10 | Glycin/NaOH |
| 7 | $KH_2PO_4/K_2PO_4$ | 11 | Glycin/NaOH |

Meßansatz (30 °C):

| | | |
|---|---|---|
| 870 | µl | der jeweils in Tabelle 3 genannten Puffersysteme |
| 20 | µl | NADH 10 mM (8,6 mg/ml Wasser) |
| 10 | µl | Enzym verdünnt |

Es wurde etwa 2 bis 3 min inkubiert, danach erfolgte die Zugabe von

| | | |
|---|---|---|
| 100 | µl | Substratlösung (100mM Ethyl-4-chlor-3-oxobuttersäure) |

Zur Bestimmung des pH-Optimum wurde die enzymatische Reaktion in dem jeweiligen in Tabelle 3 aufgeführten Puffer bestimmt. Zur Bestimmung des pH-Optimums für die Oxidationsreaktion wurde als Cofaktor NADH und als Substrat (S)-Methyl-3-hydroxybuttersäure eingesetzt. Dabei konnte fürdas erfindungsgemäße Enzym ein pH-Optimum von 6,5 bis 7 für die Reduktionsreaktion und von 7,8 bis 8,2 für die Oxidationsreaktion ermittelt werden.

5.2 pH-Stabilität

Die Bestimmung der Aktivität der rekombinanten Oxidoreduktase wurde durch Lagerung in den in Tabelle 3 genannten Puffersystemen untersucht. Dazu wurden die verschiedene Puffer (50mM) im Bereich von pH 4 bis 11 angesetzt und die gemäß Beispiel 4 hergestellte Oxidoreduktase damit verdünnt. Nach 30, 60 und 300 min Inkubation wurden aus dem Ansatz 10 µl entnommen und im Aktivitätstest gemäß Beispiel 1 eingesetzt.

Ausgangswert ist dabei der Messwert, den man unmittelbar nach Verdünnung (1:20) des Enzyms in Kaliumphosphatpuffer 50 mM pH = 7.0 erhielt. Dieser Wert entsprach unter den vorgegeben Bedingungen einer Extinktionsänderung von etwa 0,70 /min und wurde als 100%-Wert gesetzt und alle folgenden Messwerte wurden zu diesem Wert ins Verhältnis gesetzt.

Dabei wurde festgestellt, dass die rekombinante Oxidoreduktase aus P. capsulata bei einem pH von 5,5 bis 8,5 stabil ist und für mindestens 5 h ohne wesentlichen Aktivitätsverlust inkubiert werden kann. Inkubationen bei pH-Werten über 9,0 und unter 5,0 führten zu einer sofortigen Desaktivierung des Enzyms.

5.3 Temperatur-Optimum

Zur Bestimmung der optimalen Testtemperatur wurde die Enzymaktivität im Temperaturbereich von 15 °C bis 70 °C im Standardmeßansatz gemessen. Wie aus Tabelle 4 ersichtlich hat das Enzym seine maximale Aktivität bei 45°C, anschließend sinkt die Aktivität rapide ab.

Tabelle 4

| Temperatur (°C) | Aktivität in U/ml unverdünntes Enzym | Temperatur (°C) | Aktivität in U/ml unverdünntes Enzym |
|---|---|---|---|
| 15 | 73 | 45 | 176 |
| 20 | 83 | 50 | 122 |
| 25 | 128 | 55 | 45 |
| 30 | 135 | 60 | 0 |
| 35 | 163 | 65 | 0 |
| 40 | 170 | 70 | 0 |

5.4 Temperatur-Stabilität

In analoger Weise wie unter Beispiel 5.2 beschrieben wurde die Temperaturstabilität für den Bereich von 15 °C bis 70 °C bestimmt. Dazu wurde jeweils eine 1:20 Verdünnung der gereinigten Oxidoreduktase für 60 min und 180 min bei der jeweiligen Temperatur inkubiert und anschließend bei 30 °C mit dem obigen Testansatz gemessen. Auch hier wurde als Ausgangswert der Meßwert herangezogen, den man unmittelbar nach Verdünnung der gereinigten Oxidoreduktase in Kaliumphosphatpuffer 50 mM bei pH = 7,0 erhält. Dieser Wert wurde auch in diesem Beispiel als 100%-Wert gesetzt.

Das Enzym ist dabei in einem Temperaturbereich von 15°C bis 40 °C vollkommen stabil und zeigt nach 3 h Inkubation keinerlei Aktivitätsverlust. Bei 55°C ist bereits nach 30 min keine Enzymaktivität mehr nachweisbar.

5.5 Substratspektrum/Enantiomerenüberschuss

Das Substratspektrum der erfindungsgemäßen Oxidoreduktase wurde durch Messung der Enzymaktivität mit einer Reihe von Ketonen, Oxosäuren und deren Estern bestimmt. Dazu wurde der Standard Messansatz gemäß Beispiel 5.1 mit unterschiedlichen Substraten verwendet Die Aktivität mit Ethyl-4-chloracetoacetat wurde gleich 100% gesetzt und alle anderen Substrate wurden dazu ins Verhältnis gesetzt.

Für die ee-Wert-Bestimmung wurden für ausgewählte Substrate folgender Reaktionsansatz verwendet.

| | | |
|---|---|---|
| 100 | µl | NADH (50 mM) |
| 60 | µl | Substrat (100 mM) |
| + 1 bis 2 U der erfindungsgemäßen Oxidoreduktase | | |

Die Ansätze zur ee- Bestimmung wurden nach 24 h mit Chloroform extrahiert und mittels GC wurde der Enantiomerenüberschuss des resultierenden Alkohols bestimmt.

Tabelle 5

| Substrat | Relative Aktivität % | Stereoseselektivität | Substrat | Relative Aktivität % | Stereoselektivität |
|---|---|---|---|---|---|
| Ketone | | | 3-Oxosäureester | | |
| 1-Phenyl-2-propanon | 24 | 97 % S | Ethyl-4-Chloroacotoacetat | 100 | 99 % R |
| 2-chloro-1-(3-chlorpheny)ethan-1-on | 21 | 100 % R | Methyl acetoacetat | 150 | 97 % S |
| Acetophenon | 4 | n.b. | Ethyl-8-chloro-6-oxooctansäure | 20 | 100 % R |
| Caprylophenon | 0 | n.b. | Dimethyl-3-Oxo-1,8-octandioicacid | 3,5 | n.b. |
| 2-Octanon | 88 | 100 % S | Ethyl-3-oxovaleriat | 67 | n.b. |
| 3-Octanon | 30 | n.b. | Ethylacetoacetat | 100 | 99 % S |
| 2-Butanon | 99 | 50 % S | | | |

(fortgesetzt)

| Substrat | Relative Aktivität % | Stereoseselektivität | Substrat | Relative Aktivität % | Stereoselektivität |
|---|---|---|---|---|---|
| 4-Hydroxy-2-butanon | 99 | 90 % S | | | |
| Ethyl-2-oxovateriat | 41 | 97 % S | 2-Oxovaleriansäure | 0 | n.b. |
| Ethyl-2-oxo-4-phenylbuttersäure | 76 | 95 % S | 2-Oxo-3-phenyl propionsäure | 0 | n.b. |
| Ethylpyruvat | 10 | 100 % S | 2-Oxobuttersäure | 0 | n.b. |
| Ethylphenylglyoxylat | 5,2 | 100 % R | | | |

n.b. bedeutet nicht bestimmt

Wie aus Tabelle 5 ersichtlich werden von der erfindungsgemäßen Oxidoreduktase ein breites Spektrum 2- und 3- Oxosäureester sowie aromatische und aliphatische Ketone stereoselektiv reduziert.

5.5 Lösungsmittelstabilität

Die Enzymstabilität der Oxioreduktase aus P. capsulata wurde in Anwesenheit von organischen Lösungsmitteln untersucht. Dazu wurde die Oxidoreduktase jeweils 1: 20 mit den angegebenen Lösungsmittelgemischen (bei wasserrmischbaren organischen Lösungsmitteln) verdünnt und bei Raumtemperatur (20°C bis 24 °C; RT) inkubiert. Anschließend wurden 10 μl der Enzymlösung im Standardtestansatz eingesetzt. Auch hier wurde der Ausgangswert nach Verdünnung im Puffer (Kaliumphosphatpuffer (KPP) 100 mM, pH = 7.0) gleich 100 % gesetzt und alle weiteren Werte zu diesem ins Verhältnis gesetzt.

Bei den nicht mit Wasser mischbaren organischen Lösungsmittel erfolgte die Verdünnung ebenfalls in Kaliumphosphatpuffer, es wurde das gleiche Volumen organisches Lösungsmittel zum Ansatz gegeben und der Ansatz wurde bei RT im Thermomixer mit 170 rpm inkubiert. Die Aktivitätsmessung erfolgte aus der wässrigen Phase. Tabelle 6 zeigt die Ergebnisse.

Tabelle 6

| Aktivität | 8 h | 24 h | Aktivität | 8 h | 24 h |
|---|---|---|---|---|---|
| Puffer KPP 100 mM pH =7 1 mM $MgCl_2$ | 100 % | 100 % | Ethylacetat | 2 | 0 |
| 5 % Isopropanol | 77 % | 77 % | Butylacetat | 53 % | 28 % |
| 10% Isopropanol | 72 % | 72 % | Diethylether | 100 % | 100 % |
| 20 % Isopropanol | 46 % | 46 % | Methyl-tert-butylether | 100% | 100 % |
| 30 % Isopropanol | 18 % | 18 % | Diisopropylether | 100 % | 100 % |
| 5 % EtOH | 77 % | 77 % | Chloroform | 51 % | 0 % |
| 10% EtOH | 77 % | 77 % | Hexan | 60 % | 20 % |
| 20 % EtOH | 100 % | 100 % | Heptan | 74 % | 43 % |
| 30 % EtOH | 64 % | 64 % | Cyclohexan | 85 % | 70 % |
| 10 % DSMO | 42 % | 45 % | | | |
| 20 % DSMO | 30 % | 30 % | | | |

EtOH bedeutet Ethanol; DSMO bedeutet Dimethylsulfoxid

Wie aus Tabelle 6 ersichtlich zeigt die Oxidoreduktase aus P. capsulata eine erstaunliche Stabilität gegenüber organischen Lösungsmitteln. Entgegen der gängigen Lehrmeinung wird die erfindungsgemäße Oxidoreduktase in organischen wassermischbaren, sowie wasserunmischbaren Lösungsmitteln sogar stabilisiert verglichen mit Inkubation im reinen Puffer. Die erfindungsgemäße Oxidoreduktase kann daher in einem Zwei- Phasensystem, wie es in der DE 101 19274 A1 beschrieben ist, eingesetzt werden.

5.7 Präparative Umsetzungen

5.7.1 Reduktion von Ethyl-4-Chloracetoacetat zu Ethyl-(R)-4-chlor-3- hydroxybuttersäure

Für den präperativen Ansatz wurde ein Gemisch aus 34 L Puffer (100mM TEA, pH = 7,1 mM $ZnCl_2$, 10 % Glycerin), 4 L Isopropanol, 4 L 4-Chloracetoacetat, 4 g NAD und 3,6 Millionen U rekombinante Oxidoreduktase aus Pichia capsulata für 24 h bei Raumtemperatur unter ständiger Durchmischung inkubiert. Nach 24 h waren 99% des eingesetzten 4-Chloracetoacetat reduziert. Das Reaktionsgemisch wurde anschließend mit Ethylacetat extrahiert, das Lösungsmittel mittels Rotationsverdampfer entfernt und das Rohprodukt wurde destilliert. Auf diese Weise wurden 2,8 L (3,4 kg) Ethyl-(R)-4-chlor-3-hydroxybuttersäure mit einem Enantiomerenüberschuss von 99 % gewonnen. Dies entspricht einer Ausbeute von 70 % bezogen auf die eingesetzte Eduktmenge.

5.7.2. Reduktion von 2-Chlor-1-(3-chlorphenyl)ethan-1-on zu (R)-2-Chlor-1-(3-chlorphenyl)ethan-1-ol

Für den präperativen Ansatz wurde ein Gemisch aus 164 ml Puffer (100 mM TEA, pH = 7, 1 mM $ZnCl_2$, 20 % Glycerin), 16 ml Isopropanol, 20 g 2-Chlor-1-(3-chlorphenyl)ethan-1-on gelöst in 20 ml Methyl-tert-Butylether (MTBE), 10 mg NAD und 20 000 U rekombinante Oxidoreduktase aus Pichia capsulata für 24 h bei Raumtemperatur unter ständiger Durchmischung inkubiert. Nach 24 h waren 96 % des eingesetzten 2-Chlor-1-(3-chlorphenyl)ethan-1-on’s reduziert. Das Reaktionsgemisch wurde anschließend mit Ethylacetat extrahiert und das Lösungsmittel wurde mit dem Rotationsverdampfer entfernt. Auf diese Weise wurden 15 g (R)-2-Chlor-1-(3-chlorphenyl)ethan-1-ol mit einem Enantiomerenüberschuss von 100 % gewonnen. Dies entspricht einer Ausbeute von 77 % bezogen auf die eingesetzte Eduktmenge.

SEQUENCE LISTING<110> Juelich Enzyme Products

GmbH<120> Oxidoreduktase aus Pichia capsulata<130> (TH)4090<160> 10 <170> PatentIn version 3.1<210> 1<211> 1175<212> DNA<213> Pichia capsulata<400> 1

```
cgcggtggcg gccgctctag aactagtgga tcccccgggc tgcaggaatt cggcacgagg    60
atctttctca actacaatgt ctgctctctc caaaacccag gccggttaca tcttcaagaa   120
gggtgccggt cacatcgtca aggccgaggt tccaatcccc aagccaactg gtgcccaatc   180
tcttcttagg gtcaaggctg caggaatgtg ccactctgac ttgcacgtca ttggagaaac   240
attggaggtc cctaccgatg ggtacgtgct cggtcacgaa attgctggtg aattggtgga   300
gatcggagac tcggtcaacc ctgaagtttt taaggtggga ggccgttatg ctgttcatgg   360
actgaattcg tgtggatcct gtgagatgtg tcgtaccggt catgacaatg actgtactgg   420
aaatgaatcg aaatggtacg gtctgggaat tagtggtggt taccagcagt acctgctggt   480
gccaaattcg caccatctat tgcctattcc agataacgtg tcctacgaag ttgctgctgc   540
cacctctgat gctgtcttga ctccatacca tgctatcaag aattccggag tgactccatc   600
ttctaaggtg ttgatgtttg gtctgggtgg tttgggatcg aacgcacttc agatcctcaa   660
ggcatttgga gcctatgtgg ttgccgttga tgtcaagccc gcatccaaag caattgccga   720
cgaattcaaa gcggatgaat tctataccga tatcagccaa tcttcttgga aaccagcctc   780
gtttgattac tgttttgact tcgtttcgct gcaggtcacc ttcgacatct gccagaagta   840
tatcaagtcc cacggtacca tcttcccagt gggtctgggc tcgagcaagc tgactttcga   900
cttgggaaac ctggcattgc gtgaagtaaa aattgttggt aacttctggg gtacttctca   960
ggaacagatc gaagcaatgg agctggttag ctcgggtagg gtcaagcctc aagttcacac  1020
caccgaactt gaaaaccttc ctgaatcact tgaaaaactg gaggagggta agatcaatgg  1080
aagattggtt atgcttccat gatcacaaac tatttataac gagatacgag aaaaagttta  1140
atatgatgtc gtttttccaa tcaaaagggg ggccc                             1175
```

<210> 2<211> 366<212> PRT<213> Artificial<400> 2

```
Ala Val Ala Ala Ala Leu Glu Leu Val Asp Pro Pro Gly Cys Arg Asn
1               5                   10                  15

Ser Ala Arg Gly Ser Phe Ser Thr Thr Met Ser Ala Leu Ser Lys Thr
            20                  25                  30

Gln Ala Gly Tyr Ile Phe Lys Lys Gly Ala Gly His Ile Val Lys Ala
        35                  40                  45

Glu Val Pro Ile Pro Lys Pro Thr Gly Ala Gln Ser Leu Leu Arg Val
    50                  55                  60

Lys Ala Ala Gly Met Cys His Ser Asp Leu His Val Ile Gly Glu Thr
```

65 70 75 80

Leu Glu Val Pro Thr Asp Gly Tyr Val Leu Gly His Glu Ile Ala Gly
85 90 95

Glu Leu Val Glu Ile Gly Asp Ser Val Asn Pro Glu Val Phe Lys Val
100 105 110

Gly Gly Arg Tyr Ala Val His Gly Leu Asn Ser Cys Gly Ser Cys Glu
115 120 125

Met Cys Arg Thr Gly His Asp Asn Asp Cys Thr Gly Asn Glu Ser Lys
130 135 140

Trp Tyr Gly Leu Gly Ile Ser Gly Gly Tyr Gln Gln Tyr Leu Leu Val
145 150 155 160

Pro Asn Ser His His Leu Leu Pro Ile Pro Asp Asn Val Ser Tyr Glu
165 170 175

Val Ala Ala Ala Thr Ser Asp Ala Val Leu Thr Pro Tyr His Ala Ile
180 185 190

Lys Asn Ser Gly Val Thr Pro Ser Ser Lys Val Leu Met Phe Gly Leu
195 200 205

Gly Gly Leu Gly Ser Asn Ala Leu Gln Ile Leu Lys Ala Phe Gly Ala
210 215 220

Tyr Val Val Ala Val Asp Val Lys Pro Ala Ser Lys Ala Ile Ala Asp
225 230 235 240

Glu Phe Lys Ala Asp Glu Phe Tyr Thr Asp Ile Ser Gln Ser Ser Trp
245 250 255

Lys Pro Ala Ser Phe Asp Tyr Cys Phe Asp Phe Val Ser Leu Gln Val
260 265 270

Thr Phe Asp Ile Cys Gln Lys Tyr Ile Lys Ser His Gly Thr Ile Phe
275 280 285

Pro Val Gly Leu Gly Ser Ser Lys Leu Thr Phe Asp Leu Gly Asn Leu
290 295 300

Ala Leu Arg Glu Val Lys Ile Val Gly Asn Phe Trp Gly Thr Ser Gln
305 310 315 320

```
Glu Gln Ile Glu Ala Met Glu Leu Val Ser Ser Gly Arg Val Lys Pro
                325                 330                 335

Gln Val His Thr Thr Glu Leu Glu Asn Leu Pro Glu Ser Leu Glu Lys
            340                 345                 350

Leu Glu Glu Gly Lys Ile Asn Gly Arg Leu Val Met Leu Pro
        355                 360                 365
```

<210> 3<211> 17<212> DNA<213> Artificial<400> 3
gtaatacgac tataggg 17
<210> 4<211> 21<212> DNA<213> Artificial<400> 4
caattaaccc tcactaaagg g 21
<210> 5<211> 30<212> DNA<213> Artificial<400> 5
ggaattccat atgtctgctc tctccaaaac 30
<210> 6<211> 32<212> DNA<213> Artificial<400> 6
cactgcatgc tgatgtctgc tctctccaaa ac 32
<210> 7<211> 31<212> DNA<213> Artificial<400> 7
cccaagcttt catggaagca taaccaatct t 31
<210> 8<211> 1026<212> DNA<213> Pichia capsulata<400> 8

```
atgtctgctc tctccaaaac ccaggccggt tacatcttca agaagggtgc cggtcacatc      60

gtcaaggccg aggttccaat ccccaagcca actggtgccc aatctcttct tagggtcaag     120

gctgcaggaa tgtgccactc tgacttgcac gtcattggag aaacattgga ggtccctacc     180

gatgggtacg tgctcggtca cgaaattgct ggtgaattgg tggagatcgg agactcggtc     240

aaccctgaag tttttaaggt gggaggccgt tatgctgttc atggactgaa ttcgtgtgga     300

tcctgtgaga tgtgtcgtac cggtcatgac aatgactgta ctggaaatga atcgaaatgg     360

tacggtctgg gaattagtgg tggttaccag cagtacctgc tggtgccaaa ttcgcaccat     420

ctattgccta ttccagataa cgtgtcctac gaagttgctg ctgccacctc tgatgctgtc     480

ttgactccat accatgctat caagaattcc ggagtgactc catcttctaa ggtgttgatg     540

tttggtctgg gtggtttggg atcgaacgca cttcagatcc tcaaggcatt tggagcctat     600

gtggttgccg ttgatgtcaa gcccgcatcc aaagcaattg ccgacgaatt caaagcggat     660

gaattctata ccgatatcag ccaatcttct tggaaaccag cctcgtttga ttactgtttt     720

gacttcgttt cgctgcaggt caccttcgac atctgccaga agtatatcaa gtcccacggt     780

accatcttcc cagtgggtct gggctcgagc aagctgactt tcgacttggg aaacctggca     840

ttgcgtgaag taaaaattgt tggtaacttc tggggtactt ctcaggaaca gatcgaagca     900
```

atggagctgg ttagctcggg tagggtcaag cctcaagttc acaccaccga acttgaaaac 960

cttcctgaat cacttgaaaa actggaggag ggtaagatca atggaagatt ggttatgctt 1020

ccatga 1026

<210> 9<211> 341<212> PRT<213> Pichia capsulata<400> 9

```
Met Ser Ala Leu Ser Lys Thr Gln Ala Gly Tyr Ile Phe Lys Lys Gly
1               5                   10                  15

Ala Gly His Ile Val Lys Ala Glu Val Pro Ile Pro Lys Pro Thr Gly
            20                  25                  30

Ala Gln Ser Leu Leu Arg Val Lys Ala Ala Gly Met Cys His Ser Asp
        35                  40                  45

Leu His Val Ile Gly Glu Thr Leu Glu Val Pro Thr Asp Gly Tyr Val
    50                  55                  60

Leu Gly His Glu Ile Ala Gly Glu Leu Val Glu Ile Gly Asp Ser Val
65                  70                  75                  80

Asn Pro Glu Val Phe Lys Val Gly Gly Arg Tyr Ala Val His Gly Leu
                85                  90                  95

Asn Ser Cys Gly Ser Cys Glu Met Cys Arg Thr Gly His Asp Asn Asp
            100                 105                 110

Cys Thr Gly Asn Glu Ser Lys Trp Tyr Gly Leu Gly Ile Ser Gly Gly
        115                 120                 125

Tyr Gln Gln Tyr Leu Leu Val Pro Asn Ser His His Leu Leu Pro Ile
    130                 135                 140

Pro Asp Asn Val Ser Tyr Glu Val Ala Ala Ala Thr Ser Asp Ala Val
145                 150                 155                 160

Leu Thr Pro Tyr His Ala Ile Lys Asn Ser Gly Val Thr Pro Ser Ser
                165                 170                 175

Lys Val Leu Met Phe Gly Leu Gly Gly Leu Gly Ser Asn Ala Leu Gln
            180                 185                 190

Ile Leu Lys Ala Phe Gly Ala Tyr Val Val Ala Val Asp Val Lys Pro
        195                 200                 205

Ala Ser Lys Ala Ile Ala Asp Glu Phe Lys Ala Asp Glu Phe Tyr Thr
    210                 215                 220
```

```
Asp Ile Ser Gln Ser Ser Trp Lys Pro Ala Ser Phe Asp Tyr Cys Phe
225                 230                 235                 240

Asp Phe Val Ser Leu Gln Val Thr Phe Asp Ile Cys Gln Lys Tyr Ile
                245                 250                 255

Lys Ser His Gly Thr Ile Phe Pro Val Gly Leu Gly Ser Ser Lys Leu
            260                 265                 270

Thr Phe Asp Leu Gly Asn Leu Ala Leu Arg Glu Val Lys Ile Val Gly
        275                 280                 285

Asn Phe Trp Gly Thr Ser Gln Glu Gln Ile Glu Ala Met Glu Leu Val
    290                 295                 300

Ser Ser Gly Arg Val Lys Pro Gln Val His Thr Thr Glu Leu Glu Asn
305                 310                 315                 320

Leu Pro Glu Ser Leu Glu Lys Leu Glu Glu Gly Lys Ile Asn Gly Arg
                325                 330                 335

Leu Val Met Leu Pro
            340
```

<210> 10<211> 12<212> PRT<213> Pichia capsulata<400> 10

```
Lys Thr Gln Ala Gly Tyr Ile Phe Lys Lys Gly Ala
1               5                   10
```

## Patentansprüche

1. Oxidoreduktase, welche in Anwesenheit von NADH und Wasser eine Carbonylverbindung zur entsprechenden (S)-Hydroxyverbindung reduziert, **dadurch gekennzeichnet, dass** mehr als 70% der Aminosäuren identisch sind mit der Aminosäuresequenz SEQ NO:9 und sie eine spezifische Aktivität von mehr als 1 μmol pro mg Protein aufweist, bezogen auf die Umsetzung von Ethyl-4-chlor-3-oxobuttersäure zu (R)-Ethyl-4-chlor-3-hydroxybuttersäure.

2. Oxidoreduktase gemäß Anspruch 1, **dadurch gekennzeichnet, dass** 80% bis 99,5%, insbesondere 90% bis 99,5%, speziell 99% bis 99,5%, der Aminosäuren identisch zur Aminosäuresequenz SEQ ID NO:9 sind.

3. Oxidoreduktase gemäß Anspruch 2, **dadurch gekennzeichnet, dass** sie die Nukleinsäuresequenz SEQ ID NO:8 und die Aminosäuresequenz SEQ ID NO:9 aufweist.

4. Oxidoreduktase gemäß einem der Ansprüche 1 bis 3, **dadurch gekennzeichnet, dass** sie die Aminosäuresequenz SEQ ID NO:9 aufweist und ein-, zwei-, drei-, vier- oder fünffach durch ein wasserlösliches Polymer modifiziert ist.

5. Oxidoreduktase gemäß Anspruch 4, **dadurch gekennzeichnet, dass** das wasserlösliche Polymer Polyethylenglykol ist.

**6.** Oxidoreduktase gemäß Anspruch 3, **dadurch gekennzeichnet, dass** die Aminosäuresequenz SEQ ID NO:9 mit einem weiteren Polypeptid am N-terminalen oder Carboxy-terminalen Ende über eine Peptidbindung verbunden ist.

**7.** Antikörper, **dadurch gekennzeichnet, dass** er spezifisch an die Oxidoreduktase gemäß SEQ ID NO:9 bindet.

**8.** Isolierte Nukleinsäuresequenz, die für eine Oxidoreduktase gemäß Anspruch 1 kodiert, wobei die Nukleinsäuresequenz ausgewählt ist aus der Gruppe, bestehend aus:

a) einer Nukleinsäuresequenz, die SEQ ID NO:8 aufweist, oder deren komplementären Strang, und
b) einer Nukleinsäuresequenz, die mit einer Sequenz gemäß (a) unter stringenten Bedingungen hybridisiert.

**9.** Klonierungsvektor, **dadurch gekennzeichnet, dass** er eine oder mehrere der Nukleinsäure- oder DNA-Sequenzen gemäß Anspruch 8 aufweist.

**10.** Expressionsvektor, **dadurch gekennzeichnet, dass** er eine oder mehrere der Nukleinsäure- oder DNA-Sequenzen gemäß Anspruch 8 enthält und in geeigneter Weise mit einer Expressionskontrollsequenz verbunden ist.

**11.** Wirtszelle, die eine Bakterien-, Hefe-, Insekten-, Pflanzen- oder Säugetierzelle ist und mit einem Expressionsvektor gemäß Anspruch 10 transformiert oder transfektiert wurde.

**12.** Verfahren zur enantioselektiven Reduktion von Carbonylverbindungen zu den entsprechenden (S)-Hydroxyverbindungen, das **dadurch gekennzeichnet ist, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert und
b) die gebildete chirale (S)-Hydroxyverbindung isoliert wird.

**13.** Verfahren gemäß Anspruch 12, **dadurch gekennzeichnet, dass** als Carbonylverbindung eine Verbindung der Formel I

R1-C(O)-R2 (I)

eingesetzt wird, mit einem Rest R1 aus

1) -($C_1$-$C_{20}$)-Alkyl, worin Alkyl geradkettig oder verzweigt ist,
2) -($C_2$-$C_{20}$)-Alkenyl, worin Alkenyl geradkettig oder verzweigt ist und je nach Kettenlänge ein, zwei, drei oder vier Doppelbindungen enthält,
3) -($C_2$-$C_{20}$)-Alkinyl, worin Alkinyl geradkettig oder verzweigt ist und gegebenenfalls ein, zwei, drei oder vier Dreifachbindungen enthält,
4) -($C_6$-$C_{14}$)-Aryl,
5) -($C_1$-$C_8$)-Alkyl-($C_6$-$C_{14}$)-Aryl,
6) -($C_5$-$C_{14}$)-Heterocyclus, der unsubstituiert oder ein- bis dreifach substituiert ist durch Halogen, Hydroxyl, Amino oder Nitro, oder
7) -($C_3$-$C_7$)-Cycloalkyl,

wobei die unter 1) bis 7) genannten Reste R1 unsubstituiert sind oder ein-, zwei- oder dreifach substituiert sind, unabhängig voneinander, durch

a) -OH,
b) Halogen, wie Fluor, Chlor, Brom oder Jod,
c) -$NO_2$ oder
d) -$NH_2$ und

mit einem Rest R2 aus

1) -($C_1$-$C_6$)-Alkyl, worin Alkyl geradkettig oder verzweigt ist,

2) -($C_2$-$C_6$)-Alkenyl, worin Alkenyl geradkettig oder verzweigt ist und je nach Kettenlänge ein, zwei oder drei Doppelbindungen enthält,
3) -($C_2$-$C_6$)-Alkinyl, worin Alkinyl geradkettig oder verzweigt ist und gegebenenfalls ein oder zwei Dreifachbindungen enthält, oder
4) -($C_0$-$C_{10}$)-Alkyl-C(O)-O-($C_1$-$C_6$)-Alkyl, worin Alkyl gerade oder verzweigt ist und unsubstituiert oder ein- bis dreifach substituiert ist durch Halogen, Hydroxyl, Amino oder Nitro,

wobei die unter 1) bis 4) genannten Reste R2unsubstituiert sind oder ein-, zwei- oder dreifach substituiert sind, unabhängig voneinander durch

a) -OH,
b) Halogen, wie Fluor, Chlor, Brom oder Jod,
c) -$NO_2$ oder
d) -$NH_2$.

**14.** Verfahren gemäß Anspruch 12 oder 13, **dadurch gekennzeichnet, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert wird,
b) das durch die Oxidoreduktase gebildete NAD mit einem Cosubstrat zu NADH reduziert und
c) die gebildete chirale (S)-Hydroxyverbindung isoliert wird.

**15.** Verfahren gemäß Anspruch 12 oder 13, **dadurch gekennzeichnet, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert wird,
b) das durch die Oxidoreduktase gebildete NAD mit einer Dehydrogenase und einem Cosubstrat zu NADH reduziert und
c) die gebildete chirale (S)-Hydroxyverbindung isoliert wird.

**16.** Verfahren gemäß Anspruch 12 oder 13, **dadurch gekennzeichnet, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert,
b) die Reaktionen in Anwesenheit eines organischen Lösungsmittel durchgeführt und
c) die gebildete chirale (S)-Hydroxyverbindung isoliert wird.

**17.** Verfahren gemäß Anspruch 12 oder 13, **dadurch gekennzeichnet, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert wird,
b) die Reaktionen in Anwesenheit eines organischen Lösungsmittel durchgeführt,
c) das durch die Oxidoreduktase gebildete NAD mit einem Cosubstrat zu NADH reduziert, und
d) die gebildete chirale (S)-Hydroxyverbindung isoliert wird.

**18.** Verfahren gemäß Anspruch 12 oder 13, **dadurch gekennzeichnet, dass**

a) eine Carbonylverbindung in Anwesenheit der Oxidoreduktase gemäß einem oder mehreren der Ansprüche 1 bis 6, NADH und Wasser zur entsprechenden (S)-Hydroxyverbindung reduziert wird,
b) das durch die Oxidoreduktase gebildete NAD mit einer Dehydrogenase und einem Cosubstrat gleichzeitig zu NADH reduziert,
c) die Reaktionen in Anwesenheit eines organischen Lösungsmittel durchgeführt und
d) die gebildete chirale (S)-Hydroxyverbindung isoliert wird.

**19.** Verfahren gemäß Anspruch 14, 15, 17 oder 18, **dadurch gekennzeichnet, dass** als Cosubstrat Ethanol, 2-Propanol, 2-Butanol, 2-Pentanol oder 2-Octanol eingesetzt wird.

**20.** Verfahren gemäß Anspruch 15 oder 18, **dadurch gekennzeichnet, dass** als Dehydrogenase die Bäckerhefe aus Candida boidinii oder Candida parapsilosis eingesetzt wird.

**21.** Verfahren gemäß Anspruch 15 oder 18, **dadurch gekennzeichnet, dass** als . Dehydrogenase NADH abhängige Formiat-Dehydrogenase und als Cosubstrat ein Salz der Ameisensäure wie Ammoniumformiat, Natriumformiat oder Calziumformiat eingesetzt wird.

**22.** Verfahren gemäß Anspruch 16 oder 18, **dadurch gekennzeichnet, dass** als organische Lösungsmittel Diethylether, tertiär-Butylmethylether, Diisopropylether, Dibutylether, Butylacetat, Heptan, Hexan oder Cyclohexan eingesetzt werden.

**23.** Verfahren gemäß Anspruch 16 oder 18, **dadurch gekennzeichnet, dass** die organische Phase 5 % bis 80 % des gesamten Reaktionsvolumens, bevorzugt 10 % bis 40 %, beträgt.

**24.** Verfahren zur Gewinnung von chiralen (R)-Hydroxyverbindungen der Formel II,

R1-C(OH)-R2 (II)

mit einem Rest R1 aus

1) -($C_1$-$C_{20}$)-Alkyl, worin Alkyl geradkettig oder verzweigt ist,
2) -($C_2$-$C_{20}$)-Alkenyl, worin Alkenyl geradkettig oder verzweigt ist und je nach Kettenlänge ein, zwei, drei oder vier Doppelbindungen enthält,
3) -($C_2$-$C_{20}$)-Alkinyl, worin Alkinyl geradkettig oder verzweigt ist und gegebenenfalls ein, zwei, drei oder vier Dreifachbindungen enthält,
4) -($C_6$-$C_{14}$)-Aryl,
5) -($C_1$-$C_8$)-Alkyl-($C_6$-$C_{14}$)-Aryl,
6) -($C_5$-$C_{14}$)-Heterocyclus, der unsubstituiert oderein- bis dreifach substituiert ist durch Halogen, Hydroxyl, Amino oder Nitro, oder
7) -($C_3$-$C_7$)-Cycloalkyl,

wobei die unter 1) bis 7) genannten Reste R1 unsubstituiert sind oder ein-, zwei- oder dreifach substituiert sind, unabhängig voneinander, durch

a) -OH,
b) Halogen, wie Fluor, Chlor, Brom oder Jod,
c) -$NO_2$ oder
d) -$NH_2$ und

mit einem Rest R2 aus

1) -($C_1$-$C_8$)-Alkyl, worin Alkyl geradkettig oder verzweigt ist,
2) -($C_2$-$C_6$)-Alkenyl, worin Alkenyl geradkettig oder verzweigt ist und je nach Kettenlänge ein, zwei oder drei Doppelbindungen enthält,
3) -($C_2$-$C_6$)-Alkinyl, worin Alkinyl geradkettig oder verzweigt ist und gegebenenfalls ein oder zwei Dreifachbindungen enthält, oder
4) -($C_0$-$C_{10}$)-Alkyl-C(O)-O-($C_1$-$C_6$)-Alkyl, worin Alkyl gerade oderverzweigt ist und unsubstituiert oder ein- bis dreifach substituiert ist durch Halogen, Hydroxyl, Amino oder Nitro,

wobei die unter 1) bis 4) genannten Reste R2 unsubstituiert sind oder ein-, zwei- oder dreifach substituiert sind, unabhängig voneinander, durch

a) -OH,
b) Halogen, wie Fluor, Chlor, Brom oder Jod,
c) -$NO_2$ oder
d) -$NH_2$,

**dadurch gekennzeichnet ist, dass**

a) ein Gemisch, enthaltend die racemische Verbindung der Formel II, mit der Oxidoreduktase gemäß einem oder mehreren der Ansprüche 1 bis 6, NAD und Wasser inkubiert und
b) die verbliebene chirale (R)-Hydroxyverbindung der Formel II isoliert wird.

**25.** Verfahren zur Gewinnung von chiralen (R)-Hydroxyverbindungen der Formel II gemäß Anspruch 24, **dadurch gekennzeichnet ist, dass**

a) ein Gemisch, enthaltend die racemische Verbindung der Formel II, mit der Oxidoreduktase gemäß einem oder mehreren derAnsprüche 1 bis 6, NAD und Wasser inkubiert wird,
b) das durch die Oxidoreduktase gebildete NADH mit einem Cosubstrat zu NAD oxidiert und
c) die verbliebene chirale (R)-Hydroxyverbindung der Formel II isoliert wird.

**26.** Verfahren zur Gewinnung von chiralen (R)-Hydroxyverbindungen der Formel II gemäß Anspruch 24, **dadurch gekennzeichnet ist, dass**

a) ein Gemisch, enthaltend die racemische Verbindung der Formel II, mit der Oxidoreduktase gemäß einem oder mehreren der Ansprüche 1 bis 6, NAD und Wasser inkubiert wird,
b) das durch die Oxidoreduktase gebildete NADH mit einer Dehydrogenase und einem Cosubstrat zu NAD oxidiert und
c) die verbliebene chirale (R)-Hydroxyverbindung der Formel II isoliert wid.

**27.** Verfahren zur Gewinnung von chiralen (R)-Hydroxyverbindungen der Formel II gemäß Anspruch 24, **dadurch gekennzeichnet ist, dass**

a) ein Gemisch, enthaltend die racemische Verbindung der Formel II mit der Oxidoreduktase gemäß einem oder mehreren der Ansprüche 1 bis 6, NAD und Wasser inkubiert wird,
b) die Reaktionen in Anwesenheit eines organischen Lösungsmittel durchgeführt und
c) die verbliebene chirale (R)-Hydroxyverbindung der Formel II isoliert wid.

**28.** Verfahren zur Gewinnung von chiralen (R)-Hydroxy-verbindungen der Formel II gemäß Anspruch 24, **dadurch gekennzeichnet ist, dass**

a) ein Gemisch, enthaltend die racemische Verbindung der Formel II mit der Oxidoreduktase gemäß einem oder mehreren der Ansprüche 1 bis 6, NAD und Wasser inkubiert wird,
b) die Reaktionen in Anwesenheit eines organischen Lösungsmittel durchgeführt,
c) das durch die Oxidoreduktase gebildete NADH mit einer Dehydrogenase und einem Cosubstrat zu NAD oxidiert und
d) die verbliebene chirale (R)-Hydroxyverbindung der Formel II isoliert wird.

**29.** Verfahren gemäß Anspruch 25, 26 oder 28, **dadurch gekennzeichnet, dass** als Cosubstrat Aceton eingesetzt wird.

## Claims

**1.** An oxidoreductase reducing a carbonyl compound to the corresponding (S)-hydroxy compound in the presence of NADH and water, **characterized in that** more than 70% of the amino acids are identical to the amino acid sequence SEQ ID NO:9 and that it has a specific activity of more than 1 µmol per mg of protein, based on the reaction of ethyl-4-chloro-3-oxobutanoic acid to (R)-etyhl-4-chloro-3-hydroxybutanoic acid.

**2.** The oxidoreductase according to claim 1, **characterized in that** 80 % to 99.5 %, in particular 90 % to 99.5 %, especially 99 % to 99.5 %, of the amino acids are identical to amino acid sequence SEQ ID NO:9.

**3.** The oxidoreductase according to claim 2, **characterized in that** it has the nucleic acid sequence SEQ ID NO:8 and the amino acid sequence SEQ ID NO:9.

**4.** The oxidoreductase according to any of claims 1 to 3, **characterized in that** it has the amino acid sequence SEQ ID NO:9 and is modified once, twice, three, four or five times by a water-soluble polymer.

5. The oxidoreductase according to claim 4, **characterized in that** the water-soluble polymer is polyethylene glycol.

6. The oxidoreductase according to claim 3, **characterized in that** the amino acid sequence SEQ ID NO:9 is linked to a further polypeptide at the N-terminal or carboxy-terminal end via a peptide bond.

7. An antibody, **characterized in that** it specifically binds to the oxidoreductase according to SEQ ID NO:9.

8. An isolated nucleic acid sequence encoding an oxidoreductase according to claim 1, wherein the nucleic acid sequence is selected from the group consisting of:

   a) a nucleic acid sequence having SEQ ID NO:8, or the complementary strand thereof, and
   b) a nucleic acid sequence that hybridizes to a sequence according to (a) under stringent conditions.

9. A cloning vector, **characterized in that** it comprises one or several of the nucleic acid or DNA sequences according to claim 8.

10. An expression vector, **characterized in that** it comprises one or several of the nucleic acid or DNA sequences according to claim 8 and is linked in an appropriate manner to an expression control sequence.

11. A host cell which is a bacterial, yeast, insect, plant or mammalian cell and has been transformed or transfected with an expression vector according to claim 10.

12. A process for the enantioselective reduction of carbonyl compounds to the corresponding (S)-hydroxy compounds, which is **characterized in that**

   a) a carbonyl compound is reduced to the corresponding (S)-hydroxy compound in the presence of the oxidoreductase according to one or several of claims 1 to 6, NADH and water, and
   b) the chiral (S)-hydroxy compound formed is isolated.

13. The process according to claim 12, **characterized in that** a compound of Formula I

   R1-C(O)-R2 (I)

   is used as a carbonyl compound, comprising a moiety R 1 from

   1) -($C_1$-$C_{20}$)-alkyl, wherein alkyl is linear-chain or branched,
   2) -($C_2$-$C_{20}$)-alkenyl, wherein alkenyl is linear-chain or branched and, depending on the chain length, contains one, two, three or four double bonds,
   3) -($C_2$-$C_{20}$)-alkynyl, wherein alkynyl is linear-chain or branched and optionally contains one, two, three or four triple bonds,
   4) -($C_6$-$C_{14}$)-aryl,
   5) -($C_1$-$C_8$)-alkyl-($C_6$-$C_{14}$)-aryl,
   6) -($C_5$-$C_{14}$)-heterocycle, which is unsubstituted or substituted one to three times by halogen, hydroxyl, amino or nitro, or
   7) -($C_3$-$C_7$)-cycloalkyl;

   wherein the moieties R1 mentioned under 1) to 7) are unsubstituted or, independently of each other, substituted one, two or three times by

   a) -OH,
   b) halogen, such as fluorine, chlorine, bromine or iodine,
   c) -$NO_2$ or
   d) -$NH_2$,and

   comprising a moiety R2 from

   1) -($C_1$-$C_6$)-alkyl, wherein alkyl is linear-chain or branched,
   2) -($C_2$-$C_6$)-alkenyl, wherein alkenyl is linear-chain or branched and, depending on the chain length, contains one, two or three double bonds,
3) $-(C_2-C_6)$-alkynyl, wherein alkynyl is linear-chain or branched and optionally contains one or two triple bonds, or
4) $-(C_0-C_{10})$-alkyl-C(O)-O-$(C_1-C_6)$-alkyl, wherein alkyl is linear-chain or branched and is unsubstituted or substituted one to three times by halogen, hydroxyl, amino or nitro,

wherein the moieties R2 mentioned under 1) to 4) are unsubstituted or, independently of each other, substituted one, two or three times by

a) -OH,
b) halogen, such as fluorine, chlorine, bromine or iodine,
c) $-NO_2$ or
d) $-NH_2$.

**14.** The process according to claim 12 or 13, **characterized in that**

a) a carbonyl compound is reduced to the corresponding (S)-hydroxy compound in the presence of the oxidoreductase according to one or several of claims 1 to 6, NADH and water,
b) the NAD formed by the oxidoreductase is reduced to NADH with a cosubstrate, and
c) the chiral (S)-hydroxy compound formed is isolated.

**15.** The process according to claim 12 or 13, **characterized in that**

a) a carbonyl compound is reduced to the corresponding (S)-hydroxy compound in the presence of the oxidoreductase according to one or several of claims 1 to 6, NADH and water,
b) the NAD formed by the oxidoreductase is reduced to NADH with a dehydrogenase and a cosubstrate, and
c) the chiral (S)-hydroxy compound formed is isolated.

**16.** The process according to claim 12 or 13, **characterized in that**

a) a carbonyl compound is reduced to the corresponding (S)-hydroxy compound in the presence of the oxidoreductase according to one or several of claims 1 to 6, NADH and water,
b) the reactions are carried out in the presence of an organic solvent, and
c) the chiral (S)-hydroxy compound formed is isolated.

**17.** The process according to claim 12 or 13, **characterized in that**

a) a carbonyl compound is reduced to the corresponding (S)-hydroxy compound in the presence of the oxidoreductase according to one or several of claims 1 to 6, NADH and water,
b) the reactions are carried out in the absence of an organic solvent,
c) the NAD formed by the oxidoreductase is reduced to NADH with a cosubstrate, and
d) the chiral (S)-hydroxy compound formed is isolated.

**18.** The process according to claim 12 or 13, **characterized in that**

a) a carbonyl compound is reduced to the corresponding (S)-hydroxy compound in the presence of the oxidoreductase according to one or several of claims 1 to 6, NADH and water,
b) the NAD formed by the oxidoreductase is simultaneously reduced to NADH with a dehydrogenase and a cosubstrate,
c) the reactions are carried out in the presence of an organic solvent, and
d) the chiral (S)-hydroxy compound formed is isolated.

**19.** The process according to claim 14, 15, 17 or 18, **characterized in that** ethanol, 2-propanol, 2-butanol, 2-pentanol or 2-octanol is used as the cosubstrate.

**20.** The process according to claim 15 or 18, **characterized in that** baker's yeast from Candida boidinii or Candida parapsilosis is used as the dehydrogenase.

**21.** The process according to claim 15 or 18, **characterized in that** NADH-dependent formate dehydrogenase is used as the dehydrogenase and a salt of formic acid, such as ammonium formate, sodium formate or calcium formate is used as the cosubstrate.

**22.** The process according to 16 or 18, **characterized in that** diethylether, tertiary-butyl methyl ether, diisopropyl ether, dibutyl ether, butyl acetate, heptane, hexane or cyclohexane is used as the organic solvents.

**23.** The process according to claim 16 or 18, **characterized in that** the organic phase constitutes 5 % to 80 % of the total reaction volume, preferably 10 % to 40 %.

**24.** A process for obtaining chiral (R)-hydroxy compounds of Formula II

R1-C(OH)-R2 (II)

comprising a moiety R1 from

1) -($C_1$-$C_{20}$)-alkyl, wherein alkyl is linear-chain or branched,
2) -($C_2$-$C_{20}$)-alkenyl, wherein alkenyl is linear-chain or branched and, depending on the chain length, contains one, two, three or four double bonds,
3) -($C_2$-$C_{20}$)-alkynyl, wherein alkynyl is linear-chain or branched and optionally contains one, two, three or four triple bonds,
4) -($C_6$-$C_{14}$)-aryl,
5) -($C_1$-$C_8$)-alkyl-($C_6$-$C_{14}$)-aryl,
6) -($C_5$-$C_{14}$)-heterocycle, which is unsubstituted or substituted one to three times by halogen, hydroxyl, amino or nitro, or
7) -($C_3$-$C_7$)-cycloalkyl,

wherein the moieties R1 mentioned under 1) to 7) are unsubstituted or, independently of each other, substituted one, two or three times by

a) -OH,
b) halogen, such as fluorine, chlorine, bromine or iodine,
c) $-NO_2$ or
d) $-NH_2$, and

comprising a moiety R2 from

1) -($C_1$-$C_6$)-alkyl, wherein alkyl is linear-chain or branched,
2) -($C_2$-$C_6$)-alkenyl, wherein alkenyl is linear-chain or branched and, depending on the chain length, contains one, two or three double bonds,
3) -($C_2$-$C_6$)-alkynyl, wherein alkynyl is linear-chain or branched and optionally contains one or two triple bonds, or
4) -($C_0$-$C_{10}$)-alkyl-C(O)-O-($C_1$-$C_6$)-alkyl, wherein alkyl is linear-chain or branched and is unsubstituted or substituted one to three times by halogen, hydroxyl, amino or nitro,

wherein the moieties R2 mentioned under 1) to 4) are unsubstituted or, independently of each other, substituted one, two or three times by

a) -OH,
b) halogen, such as fluorine, chlorine, bromine or iodine,
c) $-NO_2$ or
d) $-NH_2$,

**characterized in that**

a) a mixture containing the racemic compound of Formula II is incubated with the oxidoreductase according to one or several of claims 1 to 6, NAD and water, and
b) the remaining chiral (R)-hydroxy compound of Formula II is isolated.

**25.** The process for obtaining chiral (R)-hydroxy compounds of Formula II according to claim 24, **characterized in that**

a) a mixture containing the racemic compound of Formula II is incubated with the oxidoreductase according to one or several of claims I to 6, NAD and water,
b) the NADH formed by the oxidoreductase is oxidized to NAD with a cosubstrate, and
c) the remaining chiral (R)-hydroxy compound of Formula II is isolated.

**26.** The process for obtaining chiral (R)-hydroxy compounds of Formula II according to claim 24, **characterized in that**

a) a mixture containing the racemic compound of Formula II is incubated with the oxidoreductase according to one or several of claims 1 to 6, NAD and water,
b) the NADH formed by the oxidoreductase is oxidized to NAD with a dehydrogenase and a cosubstrate, and
c) the remaining chiral (R)-hydroxy compound of Formula II is isolated.

**27.** The process for obtaining chiral (R)-hydroxy compounds of Formula II according to claim 24, **characterized in that**

a) a mixture containing the racemic compound of Formula II is incubated with the oxidoreductase according to one or several of claims 1 to 6, NAD and water,
b) the reactions are carried out in the presence of an organic solvent, and
c) the remaining chiral (R)-hydroxy compound of Formula II is isolated.

**28.** The process for obtaining chiral (R)-hydroxy compounds of Formula II according to claim 24, **characterized in that**

a) a mixture containing the racemic compound of Formula II is incubated with the oxidoreductase according to one or several of claims 1 to 6, NAD and water,
b) the reactions are carried out in the presence of an organic solvent,
c) the NADH formed by the oxidoreductase is oxidized to NAD with a dehydrogenase and a cosubstrate, and
d) the remaining chiral (R)-hydroxy compound of Formula II is isolated.

**29.** The process according to claim 25, 26 or 28, **characterized in that** acetone is used as the cosubstrate.

**Revendications**

**1.** Oxydoréductase, qui réduit en présence de NADH et d'eau, un composé carbonyle pour le composé (S)-hydroxy correspondant, **caractérisée en ce que** plus de 70 % des acides aminés sont identiques à la séquence d'acides aminés SEQ ID N°9 et qu'elle présente une activité spécifique de plus de 1 μmole par mg de protéine, par rapport à la réaction de l'acide éthyl-4-chloro-3-oxobutyrique en acide (R)-éthyl-4-chloro-3-hydroxybutyrique.

**2.** Oxydoréductase selon la revendication 1, **caractérisée en ce que** 80 % à 99,5 %, en particulier 90 % à 99,5 %, spécialement 99 % à 99,5 % des acides aminés sont identiques à la séquence d'acides aminés SEQ ID N°9.

**3.** Oxydoréductase selon la revendication 2, **caractérisée en ce qu'**elle présente la séquence d'acide nucléique SEQ ID N°8 et la séquence d'acides aminés SEQ ID N°9.

**4.** Oxydoréductase selon l'une des revendications 1 à 3, **caractérisée en ce qu'**elle présente la séquence d'acides aminés SEQ ID N°9 et qu'elle est modifiée une, deux, trois, quatre ou cinq fois par un polymère soluble dans l'eau.

**5.** Oxydoréductase selon la revendication 4, **caractérisée en ce que** le polymère soluble dans l'eau est le polyéthylèneglycol.

**6.** Oxydoréductase selon la revendication 3, **caractérisée en ce que** la séquence d'acides aminés SEQ ID N°9 est reliée à un autre polypeptide à l'extrémité N-terminale ou l'extrémité carboxy-terminale, par une liaison peptidique.

**7.** Anticorps **caractérisé en ce qu'**il se lie spécifiquement à l'oxydoréductase selon la SEQ ID N°9.

**8.** Séquence d'acide nucléique isolée, qui code l'oxydoréductase selon la revendication 1, où la séquence d'acide nucléique est choisie parmi le groupe consistant en :

a) une séquence d'acide nucléique, qui présente la SEQ ID N°8, ou son brin complémentaire, et

b) une séquence d'acide nucléique, qui s'hybride dans des conditions stringentes à la séquence selon (a).

**9.** Vecteur de clonage, **caractérisé en ce qu'**il présente une ou plusieurs des séquences d'acide nucléique ou d'ADN selon la revendication 8.

**10.** Vecteur d'expression, **caractérisé en ce qu'**il présente une ou plusieurs des séquences d'acide nucléique ou d'ADN selon la revendication 8 et qu'il est relié de manière appropriée à une séquence de contrôle de l'expression.

**11.** Cellule hôte, qui est une cellule de bactérie, de levure, d'insecte, de plante ou de mammifère et qui est transformée ou transfectée avec un vecteur d'expression selon la revendication 10.

**12.** Procédé de réduction énantiosélective de composés carbonyle en les composés (S)-hydroxy correspondants, **caractérisé en ce que**

a) on réduit un composé carbonyle en présence de l'oxydoréductase selon une ou plusieurs des revendications 1 à 6, de NADH et d'eau, en le composé (S)-hydroxy correspondant, et
b) le composé (S)-hydroxy chiral formé est isolé.

**13.** Procédé selon la revendication 12, **caractérisé en ce que** l'on met en oeuvre comme composé carbonyle, un composé de formule I :

R1-C(O)-R2 (I)

avec un résidu R1 choisi parmi

1) alkyle ($C_1$-$C_{20}$), où alkyle est linéaire ou ramifié,
2) alcényle ($C_2$-$C_{20}$), où alcényle est linéaire ou ramifié et contient selon la longueur de la chaîne, une, deux, trois ou quatre doubles liaisons,
3) alcynyle ($C_2$-$C_{20}$) , où alcynyle est linéaire ou ramifié et contient le cas échéant, une, deux, trois ou quatre triples liaisons,
4) aryle ($C_6$-$C_{14}$),
5) alkyl ($C_1$-$C_8$)-aryle ($C_8$-$C_{14}$),
6) hétérocycle ($C_5$-$C_{14}$), qui est non substitué ou substitué une à trois fois par halogène, hydroxyle, amino ou nitro, ou
7) cycloalkyle ($C_3$-$C_7$) ;

où les résidus R1 cités en 1) à 7) sont non substitués ou sont substitués une, deux ou trois fois, indépendamment, par

a) OH,
b) un halogène, comme le fluor, le chlore, le brome ou l'iode,
c) $NO_2$ ou
d) $NH_2$, et

avec un résidu R2 choisi parmi

1) alkyle ($C_1$-$C_6$), où alkyle est linéaire ou ramifié,
2) alcényle ($C_2$-$C_6$), où alcényle est linéaire ou ramifié et contient selon la longueur de la chaîne, une, deux ou trois doubles liaisons,
3) alcynyle ($C_2$-$C_6$), où alcynyle est linéaire ou ramifié et contient le cas échéant, une ou deux triples liaisons,
4) alkyl ($C_0$-$C_{10}$) -C(O)-O-alkyle ($C_1$-$C_6$) , où alkyle est linéaire ou ramifié et est non substitué ou substitué une à trois fois par un halogène, un hydroxyle, un amino ou un nitro,

où les résidus R2 cités en 1) à 4) sont non substitués ou sont substitués une, deux ou trois fois, indépendamment, par

a) OH,
b) un halogène, comme le fluor, le chlore, le brome ou l'iode,
c) $NO_2$ ou
d) $NH_2$.

**14.** Procédé selon la revendication 12 ou 13, **caractérisé en ce que**

a) on réduit un composé carbonyle en présence de l'oxydoréductase selon une ou plusieurs des revendications 1 à 6, de NADH et d'eau, en le composé (S)-hydroxy correspondant,
b) le NAD formé par l'oxydoréductase est réduit en NADH avec un cosubstrat, et
c) le composé (S)-hydroxy chiral formé est isolé.

**15.** Procédé selon la revendication 12 ou 13, **caractérisé en ce que**

a) on réduit un composé carbonyle en présence de l'oxydoréductase selon une ou plusieurs des revendications 1 à 6, de NADH et d'eau, en le composé (S)-hydroxy correspondant,
b) le NAD formé par l'oxydoréductase est réduit en NADH avec une déshydrogénase et un cosubstrat, et
c) le composé (S)-hydroxy chiral formé est isolé.

**16.** Procédé selon la revendication 12 ou 13, **caractérisé en ce que**

a) on réduit un composé carbonyle en présence de l'oxydoréductase selon une ou plusieurs des revendications 1 à 6, de NADH et d'eau, en le composé (S)-hydroxy correspondant,
b) la réaction est réalisée en présence d'un solvant organique, et
c) le composé (S)-hydroxy chiral formé est isolé.

**17.** Procédé selon la revendication 12 ou 13, **caractérisé en ce que**

a) on réduit un composé carbonyle en présence de l'oxydoréductase selon une ou plusieurs des revendications 1 à 6, de NADH et d'eau, en le composé (S)-hydroxy correspondant,
b) la réaction est réalisée en présence d'un solvant organique,
c) le NAD formé par l'oxydoréductase est réduit en NADH avec un cosubstrat, et
d) le composé (S)-hydroxy chiral formé est isolé.

**18.** Procédé selon la revendication 12 ou 13, **caractérisé en ce que**

a) on réduit un composé carbonyle en présence de l'oxydoréductase selon une ou plusieurs des revendications 1 à 6, de NADH et d'eau, en le composé (S)-hydroxy correspondant,
b) le NAD formé par l'oxydoréductase est réduit en NADH avec une déshydrogénase et un cosubstrat, simultanément,
c) la réaction est réalisée en présence d'un solvant organique, et
d) le composé (S)-hydroxy chiral formé est isolé.

**19.** Procédé selon la revendication 14, 15, 17 ou 18, **caractérisé en ce que** l'on met en oeuvre comme cosubstrat, l'éthanol, le 2-propanol, le 2-butanol, le 2-pentanol ou le 2-octanol.

**20.** Procédé selon la revendication 15 ou 18, **caractérisé en ce que** l'on met en oeuvre comme déshydrogénase, la levure de boulangerie de *Candida boldinii* ou *Candida parapsilosis.*

**21.** Procédé selon la revendication 15 ou 18, **caractérisé en ce que** l'on met en oeuvre comme déshydrogénase, la formate déshydrogénase dépendant de NADH et comme cosubstrat, un sel d'acide formique tel que le formate d'ammonium, le formate de sodium ou le formate de calcium.

**22.** Procédé selon la revendication 16 ou 18, **caractérisé en ce que** l'on met en oeuvre comme solvant organique, le diéthyléther, le t-butylméthyléther, le diisopropyléther, le dibutyléther, l'acétate de butyle, l'heptane, l'hexane ou le cyclohexane.

**23.** Procédé selon la revendication 16 ou 18, **caractérisé en ce que** la phase organique se situe dans l'intervalle allant de 5 % à 80 % du volume réactionnel total, de préférence de 10 % à 40 %.

**24.** Procédé d'obtention de composés (R)-hydroxy chiraux de formule II :

R1-C (OH) -R2 (II)

avec un résidu R1 choisi parmi

1) alkyle ($C_1$-$C_{20}$), où alkyle est linéaire ou ramifié,
2) alcényle ($C_2$-$C_{20}$), où alcényle est linéaire ou ramifié et contient selon la longueur de la chaîne, une, deux, trois ou quatre doubles liaisons,
3) alcynyle ($C_2$-$C_{20}$), où alcynyle est linéaire ou ramifié et contient le cas échéant, une, deux, trois ou quatre triples liaisons,
4) aryle ($C_6$-$C_{14}$),
5) alkyl ($C_1$-$C_8$) -aryle ($C_8$-$C_{14}$),
6) hétérocycle ($C_5$-$C_{14}$), qui est non substitué ou substitué une à trois fois par halogène, hydroxyle, amino ou nitro, ou
7) cycloalkyle ($C_3$-$C_7$) ;

où les résidus R1 cités en 1) à 7) sont non substitués ou sont substitués une, deux ou trois fois, indépendamment, par

a) OH,
b) un halogène, comme le fluor, le chlore, le brome ou l'iode,
c) $NO_2$ ou
d) $NH_2$, et

avec un résidu R2 choisi parmi

1) alkyle ($C_1$-$C_6$), où alkyle est linéaire ou ramifié,
2) alcényle ($C_2$-$C_6$), où alcényle est linéaire ou ramifié et contient selon la longueur de la chaîne, une, deux ou trois doubles liaisons,
3) alcynyle ($C_2$-$C_6$), où alcynyle est linéaire ou ramifié et contient le cas échéant, une ou deux triples liaisons,
4) alkyl ($C_0$-$C_{10}$)-C(O)-O-alkyle ($C_1$-$C_6$) , où alkyle est linéaire ou ramifié et est non substitué ou substitué une à trois fois par halogène, hydroxyle, amino ou nitro,

où les résidus R2 cités en 1) à 4) sont non substitués ou sont substitués une, deux ou trois fois, indépendamment, par

a) OH,
b) un halogène, comme le fluor, le chlore, le brome ou l'iode,
c) $NO_2$ ou
d) $NH_2$,

**caractérisé en ce que**

a) on incube un mélange contenant le composé racémique de formule (II), avec l'oxydoréductase selon une ou plusieurs des revendications 1 à 6, du NAD et de l'eau, et
b) le composé (R)-hydroxy chiral formé de formule II est isolé.

**25.** Procédé d'obtention de composés (R)-hydroxy chiraux de formule II selon la revendication 24, **caractérisé en ce que** :

a) on incube un mélange contenant le composé racémique de formule (II), avec l'oxydoréductase selon une ou plusieurs des revendications 1 à 6, du NAD et de l'eau,
b) le NADH formé par l'oxydoréductase est oxydé en NAD avec un cosubstrat, et
c) le composé (R)-hydroxy chiral formé de formule II est isolé.

**26.** Procédé d'obtention de composés (R)-hydroxy chiraux de formule II selon la revendication 24, **caractérisé en ce que** :

a) on incube un mélange contenant le composé racémique de formule (II), avec l'oxydoréductase selon une ou plusieurs des revendications 1 à 6, du NAD et de l'eau,
b) le NADH formé par l'oxydoréductase est oxydé en NAD avec une déshydrogénase et un cosubstrat, et
c) le composé (R)-hydroxy chiral formé de formule II est isolé.

**27.** Procédé d'obtention de composés (R)-hydroxy chiraux de formule II selon la revendication 24, **caractérisé en ce que** :

a) on incube un mélange contenant le composé racémique de formule (II), avec l'oxydoréductase selon une ou plusieurs des revendications 1 à 6, du NAD et de l'eau,
b) la réaction est réalisée en présence d'un solvant organique, et
c) le composé (R)-hydroxy chiral formé de formule II est isolé.

**28.** Procédé d'obtention de composés (R)-hydroxy chiraux de formule II selon la revendication 24, **caractérisé en ce que** :

a) on incube un mélange contenant le composé racémique de formule (II), avec l'oxydoréductase selon une ou plusieurs des revendications 1 à 6, du NAD et de l'eau,
b) la réaction est réalisée en présence d'un solvant organique,
c) le NADH formé par l'oxydoréductase est oxydé en NAD avec une déshydrogénase et un cosubstrat, et
d) le composé (R)-hydroxy chiral formé de formule II est isolé.

**29.** Procédé selon la revendication 25, 26 ou 28, **caractérisé en ce que** l'on met en oeuvre comme cosubstrat, l'acétone.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5523223 A
- US 5763236 A
- DE 10119274 A1


### In der Beschreibung aufgeführte Nicht-Patentliteratur


- *Enzyme Engineering,* 1982, vol. 6, 107
- Alcohol dehydrogenases. The Enzymes. New York: Academic Press, 1963, 25-83
- *Biosci. Biotechnol. Biochem.,* 1999, vol. 63 (10), 1721-1729
- *Biochim,. Biophys. Acta,* 1982, vol. 716, 298-307
- *FEMS Microbiology Letters,* 1999, vol. 170, 31-39
- *J. Biotechnol. Bioeng.,* 1999, vol. 64, 187-193
- **Sambrok ; Russel.** Molecular Cloning a laboratory Manual. vol. 1
- **Tishkov et al.** *J. Biotechnol. Bioeng.,* 1999, vol. 64, 187-193
- **Lowry et al.** *Journal of Biological Chemistry,* 1951, vol. 193, 265-275
- **Peterson et al.** *Analytical Biochemistry,* 1979, vol. 100, 201-220